(19) Europäisches Patentamt
European Patent Office
Office européen des brevets

(11) Publication number: **0 210 545 A2**

(12) # EUROPEAN PATENT APPLICATION

(21) Application number: **86109764.0**

(22) Date of filing: **16.07.86**

(51) Int. Cl.⁴: **C 07 F 9/30**
C 07 F 9/32, C 07 F 9/65
A 61 K 31/66

(30) Priority: **24.07.85 US 758231**

(43) Date of publication of application:
**04.02.87 Bulletin 87/6**

(84) Designated Contracting States:
**CH DE FR GB IT LI NL**

(71) Applicant: **MERCK & CO. INC.**
**126, East Lincoln Avenue P.O. Box 2000**
**Rahway New Jersey 07065(US)**

(72) Inventor: **Patchett,d Arthur A.**
**1090 Minisink Way**
**Westfield New Jersey 07090(US)**

(72) Inventor: **Greenlee, William J.**
**115 Herrick Avenue**
**Teaneck New Jersey 07666(US)**

(72) Inventor: **Parsons, William H.**
**2171 Oliver Street**
**Rahway New Jersey 07065(US)**

(74) Representative: **Abitz, Walter, Dr.-Ing. et al,**
**Abitz, Morf, Gritschneder, Freiherr von Wittgenstein**
**Postfach 86 01 09**
**D-8000 München 86(DE)**

(54) **Phosphorous containing enzyme inhibitors.**

(57) Dipeptides and amino acids of the formula
A–E–G
are disclosed wherein
A is, e.g., hydrogen, or $R_a$–, $R_bCO$ or $R_bSO_2$– where $R_a$ and $R_b$ are e.g., alkyl, cycloalkyl, or aryl;

E is 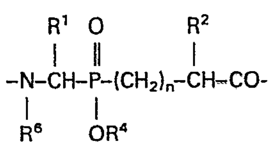 ;

G is $-R^3$ or is 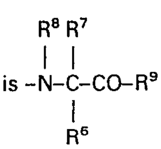 ,

wherein
$R^1$ is, e.g., alkyl;
n is 0, 1;
$R^2$ is, e.g., hydrogen or alkyl;
$R^3$ is OH, $NH_2$, $NHR^3_a$, $NR^3_aN^3_b$, $OR^3_c$ where $R^3_b$, and $R^3_c$ are e.g., alkyl;
$R^4$ is, e.g., hydrogen or alkyl
$R^6$ is H or methyl;
$R^7$ is, e.g., hydrogen or alkyl;
$R^8$ is, e.g., hydrogen, methyl or cycloalkyl such that when $R^8$ is cycloalkyl, $R^6$ and $R^7$ are hydrogen;
$R^9$ is, e.g., hydroxy.
The compounds have enzyme inhibitor activity e.g., renin inhibition angiotensin conv··rting enzyme inhibition.

EP 0 210 545 A2

Croydon Printing Company Ltd.

## TITLE OF THE INVENTION

PHOSPHOROUS CONTAINING ENZYME INHIBITORS

## BACKGROUND OF THE INVENTION

The present invention is concerned with novel compounds (substituted amino acids and dipeptides) which inhibit renin and in some instances also inhibit angiotensin converting enzyme (ACE).

The present invention is also concerned with pharmaceutical compositions containing the novel compounds of the present invention as active ingredients, with thier use for treating renin and/or ACE associated conditions, with diagnostic methods which utilize these compounds, and with processes for their preparation.

Small peptide inhibitors of renin have been reported by Kokubu et al. (Biochem. Biophys. Res. Communs, 118, 929 (1984)) and by Fehrentz et al. (Febs. Letts., 167, 273 (1984)), who described peptide aldehyde inhibitors. R. Matsuda et al. in European Patent Application 128,762 (Sankyo Co., Ltd., 1984) also disclose tripeptide aldehydes as renin inhibitors.

Some of the compounds disclosed in the present invention also inhibit angiotensin-converting enzyme. Peptides containing aminophosphonic acids as the carboxyl-terminal components, which are inhibitors of A.C.E. have previously been reported by Y. Kido et al., in J. Antibiot. (Tokyo), 37, 965-9 (1984) and in Japanese Patent J59187-790-A (Kyowa Hakko Kogyo KK) (1984). Also, phosphinylalkanoyl substituted proline analogs which inhibit A.C.E. have previously been described by E. Petrillo et al., U. S. Patents No 4,168,267; 4,337,201 (E. R. Squibb and Sons, 1979, 1982).

Compounds of no more than two amino acids or the equivalent, which incorporate phosphorous-containing elements as the carboxyl-terminal or next-to carboxyl-terminal component have been discovered. They are potent renin inhibitors and may be orally acive.

## SUMMARY OF THE INVENTION

Compounds which 1) include dipeptides and substituted amino acids and 2) contain phosphorous having renin inhibitory activity and which may be orally active.

The present invention comprises compounds of the following formula:

A-E-G

(I)

wherein:

A is hydrogen, or $R_a$-, $R_bCO$ or $R_bSO_2$- where $R_a$ and $R_b$ are alkyl, cycloalkyl, aryl, heterocyclic, heterocyclic alkyl, heterocyclic aryl, aryloxy alkyl, heterocyclic aryloxy alkyl, aryl alkyl, heterocyclic aryl alkyl, heterocyclic oxyalkyl, and $R_a$ and $R_b$ may be substituted with up to three members selected from amino, carboxy, alkoxy carbonyl, hydroxy, alkyl, halo and alkoxy groups.

E is 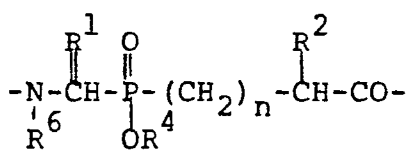 ;

G is $-R^3$ or is 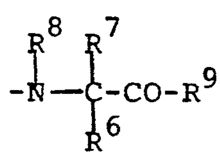 ,

wherein

$R^1$ is alkyl, alkenyl, alkynyl, cycloalkyl, cycloalkenyl, cycloalkyl alkyl, aryl alkyl, heterocyclic, heterocyclic alkyl, heterocyclic aryl alkyl, each of which may be substituted with up to three members selected from alkyl, halo, amino and alkoxy groups.

n is 0, 1.

$R^2$ is hydrogen, alkyl, alkenyl, alkynyl, cycloalkyl, cycloalkenyl, cycloalkyl alkyl, aryl, aryl alkyl, heterocyclic, heterocyclic alkyl, each of which may be substituted with up to three members selected from alkyl, hydroxy, halo, amino, alkylamino, dialkylamino, and alkoxy groups.

$R^3$ is OH, $NH_2$, $NHR^3_a$, $NR^3_aN^3_b$, $OR^3_c$ where $R^3_a$, $R^3_b$, and $R^3_c$ are separately alkyl, alkenyl, alkynyl, cycloalkyl, cycloalkenyl, cycloalkyl alkyl, aryl, aryl alkyl, heterocyclic, heterocyclic alkyl, each of which may be substituted with up to three groups selected from amino, alkylamino, dialkyl amino, trialkyl ammonium, hydroxy, alkoxy, aryloxy, aryl alkoxyl, or halo; $R^3_c$ may also be $R^3_d$-CO-V-$CR^3_eR^3_f$ wherein $R^3_d$ is alkyl or aryl; $R^3_e$ and $R^3_f$ are hydrogen or alkyl; and V is -O- or -NH-.

$R^4$ is hydrogen, alkyl, alkenyl, alkynyl, aryl, arylalkyl, each of which may be substituted with up to three members selected from amino, alkylamino, dialkylamino, trialkyl ammonium, hydroxy, alkoxy, halo or alkyl groups; or $R^4_a$-CO-V-$CR^4_bR^4_c$ wherein $R^4_a$ is alkyl or aryl; $R^4_b$ and $R^4_c$ are hydrogen or alkyl; and V is -O- or -NH-.

$R^6$ is H or methyl.

$R^7$ is hydrogen, alkyl, alkenyl, alkynyl, cycloalkyl cycloalkenyl, aryl, cycloalkyl alkyl, aryl alkyl, heterocyclic, heterocyclic alkyl, aryloxy alkyl, heterocyclic oxy alkyl,

heterocyclic oxy, each of which may be substituted with up to three groups selected from amino, alkyl amino, dialkyl amino, trialkyl ammonium, hydroxy, alkoxy, carboxy, alkoxycarbonyl, alkylthio, arylthio, thiol, guanidino, carboxamido and alkanoylamino,

and when taken together with $NR^8$ may be a cyclic amino acid of the formula:

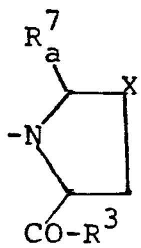 , 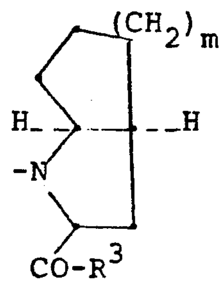 or 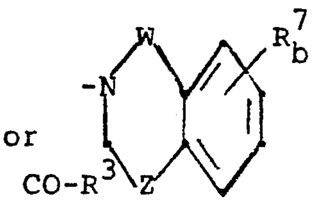

where $R^7_a$ is hydrogen, phenyl, hydroxyphenyl; X is -S- or $-CH_2-$ or $-CH-R^7_b$; m is 1 or 2; and $R^7_b$ is cyclohexyl, phenylthio; W and Z are single bonds or $-CH_2-$, and $R^3$ is as defined above.

$R^8$ is hydrogen, methyl and cycloalkyl (including cyclopentyl and indanyl) such that when $R^8$ is cycloalkyl, $R^6$ and $R^7$ are hydrogen.

$R^9$ is hydroxy, $OR^3_a$, $-NH_2$, $-NHR^3_a$, $NR^3_aR^3_b$, where $R^3_a$, $R^3_b$ and $R^3_c$ are as defined above such that when A is absent, $R^9$ can be 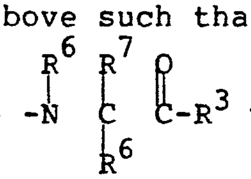 where $R^3$,

1 $R^6$ and $R^7$ are as defined above.

In the above definitions, the terms alkyl, alk, alkenyl, and alkynyl include hydrocarbon groups having up to 8 carbon atoms which groups may be straight chain or branched chain. Preferred alkyl or alk groups have 1-4 carbon atoms. Preferred alkenyl and alkynyl groups have 3 to 6 carbon atoms.

The term halo means fluoro, chloro, bromo and iodo.

The aryl substituent is a hydrocarbon having 6-12 carbon atoms exemplified by phenyl, naphthyl, biphenyl and cycloalkyl-fused derivatives thereof such as indanyl and tetralinyl.

The heterocyclic substituents recited above represents any 5- or 6-membered ring containing from one to three heteroatoms selected from the group consisting of nitrogen, oxygen and sulfur wherein the nitrogen and sulfur heteroatoms may optionally be quaternized, and including any bicyclic group in which any of the above heterocyclic rings is fused to a benzene ring. Heterocyclic substituents in which nitrogen is the heteroatom are preferred; and of these, those containing a single nitrogen atom are preferred. Fully saturated and fully unsaturated heterocyclic substituents are also preferred. Thus, piperidine is a preferred saturated heterocyclic substituent. Other preferred heterocyclic substituents are pyrrolidinyl, imidazolidinyl, morpholinyl, tetrahydrofuranyl thienyl, pyrimidinyl, imidazolyl, indolyl, quinolinyl, isoquinolinyl and benzothieny groups and the like.

Cycloalkyl and cycloalkenyl groups contain 3 to 12 carbon atoms and may be bridged. They are exemplified by cyclopropyl, cyclopentyl, cyclohexyl,

cyclohexenyl, nobornenyl, adamantyl, bicyclo[3.3.0]-octanyl and perhydronaphthyl groups and the like.

Preferred A units include benzyloxycarbonyl, t-butoxycarbonyl, 1-naphthyloxyacetyl, and 1-naphthylacetyl.

Preferred E units include those with the following formulae:

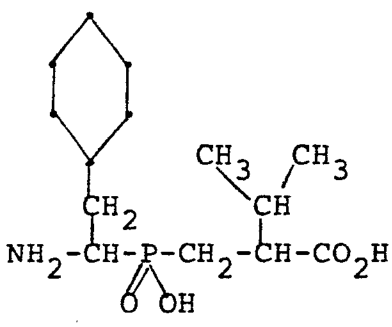

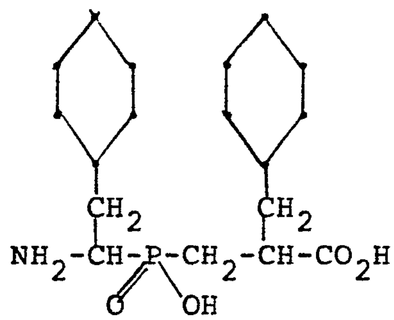

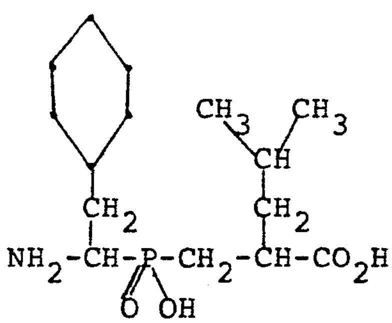

or

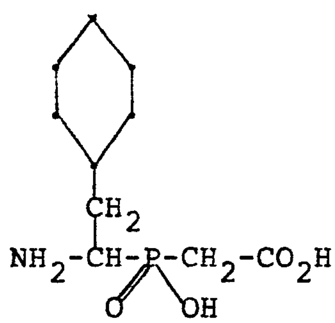

Preferred G substituents include -OH, -OEt, $-NH_2$.

The compounds disclosed have asymmetric centers and occur as racemates, racemic mixtures and as individual diastereomers. All isomeric forms are

included in the present compounds as appropriate. The stereocenters present in the E unit of the peptides of formula I are in general of the chirality which corresponds to the naturally-occurring (L) amino acids. Thus, for example, the unit E of the formula below possesses the stereochemistry shown in the preferred form:

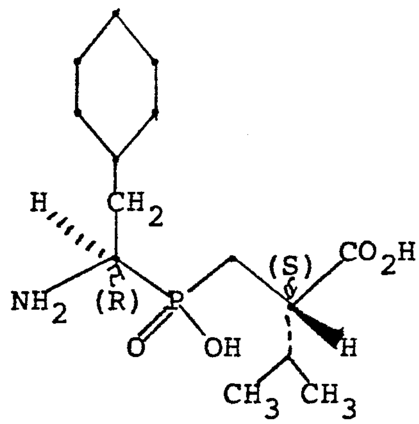

The following are illustrative examples of peptides of the present invention:

1. (N-carbobenzoxy-1-amino-2-cyclohexylethyl) 2-carboxy-4-methylpentylphosphinic acid
2. (N-carbobenzoxy-1-amino-2-cyclohexylethyl) 2-carboxy-3-methylbutylphosphinic acid
3. (N-t-butoxycarbonyl-1-amino-2-cyclohexylethyl) 2-carboxy-3-methylbutylphosphinic acid
4. (N-carbobenzoxy-1-amino-2-cyclohexylethyl) 2-carbomethoxy-4-methylpentylphosphinic acid
5. (N-carbobenzoxy-1-amino-2-cyclohexylethyl) 2-carboxamido-4-methylpentylphosphinic acid
6. (N-t-butoxycarbonyl-1-amino-2-cyclohexylethyl) 2-(N-benzyl)carboxamido-3-methylbutylphosphinic acid

7. [N-(2-phenyl)ethyl-1-amino-2-cyclohexylethyl] 2-carboxy-4-methylpentylphosphinic acid

8. [N-(N-benzyl)carboxamidomethyl-1-amino-2-cyclohexylethyl] 2-carboxy-3-methylbutylphosphinic acid

9. [N-(1-carboethoxy-2-phenylethyl)-1-amino-2-cyclohexylethyl] 2-carboxy-3-methylbutylphosphinic acid

10. (N-carbobenzoxy-1-amino-2-cyclohexylethyl)carbomethoxymethylphosphinic acid

11. (N-carbobenzoxy-1-amino-2-cyclohexylethyl)carboxymethylphosphinic acid

12. (N-carbobenzoxy-1-amino-2-cyclohexylethyl) carboxamidomethylphosphinic acid

13. (N-carbobenzoxy-1-amino-2-cyclohexylethyl) 2-carboxy-3-cyclohexylpropylphosphinic acid

14. [N-(3-phenyl)propionyl-1-amino-2-cyclohexylethyl] 2-carboxy-4-methylpentylphosphinic acid

15. (N-phenoxyacetyl-1-amino-2-cyclohexylethyl) 2-carboxy-3-methylbutylphosphinic acid

16. [N-(4-amino)butanoyl-1-amino-2-cyclohexylethyl] 2-carboxy-3-methylbutylphosphinic acid

17. (N-naphthyloxyacetyl)-1-amino-2-cyclohexylethyl) 2-carboxy-3-methylbutylphosphinic acid

18. Methyl (N-carbobenzoxy-1-amino-2-cyclohexylethyl) 2-carboxy-4-methylpentylphosphinate

19. Methyl (N-carbobenzoxy-1-amino-2-cyclohexylethyl)-carbomethoxymethylphosphinate

20. Ethyl [N-(3-phenyl)propionyl-1-amino-2-cyclohexylethyl] 2-carbomethoxy-3-methylbutylphosphinate

21. [N-(1-carboethoxy-5-aminopentyl)-1-amino-2-cyclohexylethyl] 2-carboxy-3-methylbutylphosphinic acid

22. [N-(1-carbobenzoxy-5-aminopentyl)-1-amino-2-cyclohexylethyl] 2-carboethoxy-3-methylbutylphosphinic acid

23. [N-carbobenzoxy-1-amino-2-cyclohexylethyl] 2-(N-(m-aminomethyl)benzyl)carboxamido-3-methylbutylphosphinic acid

Another group of illustrative compounds are:

24. (N-CBZ-1-amino-2-cyclohexylethyl) 2-carboxy-4-methylpentylphosphinic acid
25. (N-CBZ-1-amino-2-cyclohexylethyl) 2-carbomethoxy-4-methylpentylphosphinic acid
26. Methyl (N-CBZ-1-amino-2-cyclohexylethyl) 2-carbomethoxy-4-methylpentylphosphinate
27. Methyl (N-BOC-1-amino-2-cyclohexylethyl) 2-carbomethoxy-4-methylpentylphosphinate
28. (1-Amino-2-cyclohexylethyl) 2-carbomethoxy-4-methylpentylphosphinic acid hydrobromide
29. Methyl (1-amino-2-cyclohexylethyl) 2-carbomethoxy-4-methylpentylphosphinate hydrobromide
30. Trimethylacetoxymethyl (N-CBZ-1-amino-2-cyclohexylethyl) 2-carbomethoxy-4-methylpentylphosphinate
31. Methyl (N-BOC-1-amino-2-cyclohexylethyl) 2-carbomethoxy-4-methyl-(E)-2-pentenylphosphinate
32. Methyl (N-BOC-1-amino-2-cyclohexylethyl) 2-carbomethoxy-4-methyl-(Z)-2-pentenylphosphinate
33. (N-BOC-1-Amino-2-cyclohexylethyl) 2-carboxy-4-methyl-(E)-2-pentenylphosphinic acid
34. (N-BOC-1-Amino-2-cyclohexylethyl) 2-carboxy-4-methyl-(Z)-2-pentenylphosphinic acid
35. Methyl (N-CBZ-1-amino-2-cyclohexylethyl) 2-carbomethoxy-3-cyclohexylphosphinate
36. Methyl (N-CBZ-1-amino-2-cyclohexylethyl) carbomethoxymethylphosphinate

37. (1-Amino-2-cyclohexylethyl) carbomethoxymethylphosphinic acid
38. Methyl (1-amino-2-cyclohexylethyl)carbomethoxymethylphosphinate hydrochloride
39. Methyl (N-CBZ-1-amino-3-methylbutyl)carbomethoxymethylphosphinate
40. Methyl (N-CBZ-1-amino-2-phenylethyl) 2-carbomethoxy-3-phenylpropylphosphinate
41. (1-Amino-2-phenylethyl) 2-carbomethoxy-3-phenylpropylphosphinate

The Formula I compounds include many which bear acidic and/or basic groups. Pharmaceutically acceptable salts of these formula I compounds are also included. Among useful the acid addition salts are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Salts of the bases include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such

as arginine, lysine, and so forth. Also, the basic nitrogen-containing groups can be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others. Water or oil-soluble or dispersible products are thereby obtained. Conventional processes are used to prepare the pharmaceutically acceptable salts.

The present invention is also directed to combinations of the novel renin-inhibitory compounds of Formula I with one or more antihypertensive agents selected from the group consisting of diuretics, α and/or ß-adrenergic blocking agents, CNS-acting anti-hypertensive agents, adrenergic neuron blocking agents, vasodilators, angiotensin I converting enzyme inhibitors, calcium channel blockers and other antihypertensive agents.

For example, the compounds of this invention can be given in combination with such compounds or salt or other derivative forms thereof as:

<u>Diuretics</u>: acetazolamide; amiloride; bendroflumethiazide; benzthiazide; bumetanide; chlorothiazide; chlorthalidone; cyclothiazide; ethacrynic acid; furosemide; hydrochlorothiazide; hydroflumethiazide; indacrinone (racemic mixture, or as either the (+) or (-) enantiomer alone, or a manipulated ratio, e.g., 9:1 of said enantiomers,

respectively); metolazone; methyclothiazide; muzolimine; polythiazide; quinethazone; sodium ethacrynate; sodium nitroprusside; spironolactone; ticrynafen; triamterene; trichlormethiazide;

α-Adrenergic Blocking Agents: dibenamine; phentolamine; phenoxybenzamine; prazosin; tolazoline;

β-Adrenergic Blocking Agents: atenolol; metoprolol; nadolol; propranolol; timolol;

((±)-2-[3-(tert-butylamino)-2-hydroxypropoxy]-2-furananilide) (ancarolol);

(2-acetyl-7-(2-hydroxy-3-isopropylaminopropoxy)benzofuran HCl) (befunolol);

((±)-1-(isopropylamino)-3-(p-(2-cyclopropylmethoxyethyl)-phenoxy)-2-propranol HCl) (betaxolol);

(1-[(3,4-dimethoxyphenethyl)amino]-3-(m-tolyloxy)-2-propanol HCl) (bevantolol);

(((±)-1-(4-((2-isopropoxyethoxy)methyl)phenoxy)-3-isopropylamino-2-propanol)fumarate) (bisoprolol);

(4-(2-hydroxy-3-[4-(phenoxymethyl)-piperidino]-propoxy)-indole);

(carbazolyl-4-oxy-5,2-(2-methoxyphenoxy)-ethylamino-2-propanol);

(1-((1,1-dimethylethyl)amino)-3-((2-methyl-1H-indol-4-yl)oxy)-2-propanol benzoate) (bopindolol);

(1-(2-exobicyclo[2.2.1]-hept-2-ylphenoxy)-3-[(1-methylethyl)-amino]-2-propanol HCl) (bornaprolol);

(o-[2-hydroxy-3-[(2-indol-3-yl-1,1-dimethylethyl)-amino]propoxy]benzonitrile HCl) (bucindolol);

(α-[(tert.butylamino)methyl]-7-ethyl-2-benzofuranmethanol) (bufuralol);

(3-[3-acetyl-4-[3-(tert.butylamino)-2-hydroxypropyl]-phenyl]-1,1-diethylurea HCl) (celiprolol);

((±)-2-[2-[3-[(1,1-dimethylethyl)amino]-2-hydroxy-propoxy]phenoxy]-N-methylacetamide HCl) (cetamolol);

(2-benzimidazolyl-phenyl(2-isopropylaminopropanol));

((±)-3'-acetyl-4'-(2-hydroxy-3-isopropylaminopropoxy)-acetanilide HCl) (diacetolol);

(methyl-4-[2-hydroxy-3-[(1-methylethyl)aminopropoxy]]-benzenepropanoate HCl) (esmolol);

(erythro-DL-1-(7-methylindan-4-yloxy)-3-isopropylamino-butan-2-ol);

(1-(tert.butylamino)-3-[O-(2-propynyloxy)phenoxy]-2-propanol (pargolol);

(1-(tert.butylamino)-3-[o-(6-hydrazino-3-pyridazinyl)-phenoxy]-2-propanol diHCl) (prizidilol);

((-)-2-hydroxy-5-[(R)-1-hydroxy-2-[(R)-(1-methyl-3-phenylpropyl)amino]ethyl]benzamide);

(4-hydroxy-9-[2-hydroxy-3-(isopropylamino)-propoxy]-7-methyl-5H-furo[3,2-g][1]-benzopyran-5-one) (iprocrolol);

((-)-5-(tert.butylamino)-2-hydroxypropoxy]-3,4-dihydro-1-(2H)-naphthalenone HCl) (levobunolol);

(4-(2-hydroxy-3-isopropylamino-propoxy)-1,2-benziso-thiazole HCl);

(4-[3-(tert.butylamino)-2-hydroxypropoxy]-N-methyliso-carbostyril HCl);

((±)-N-2-[4-(2-hydroxy-3-isopropyl aminopropoxy)-phenyl]ethyl-N'-isopropylurea) (pafenolol);

(3-[[(2-trifluoroacetamido)ethyl]amino]-1-phenoxy-propan-2-ol);

(N-(3-(o-chlorophenoxy)-2-hydroxypropyl)-N'-(4'-chloro-2,3-dihydro-3-oxo-5-pyridazinyl)ethylenediamine);

((±)-N-[3-acetyl-4-[2-hydroxy-3-[(1-methylethyl)amino]-propoxy]phenyl]butanamide) (acebutolol);

((±)-4'-[3-(tert-butylamino)-2-hydroxypropoxy]spiro-[cyclohexane-1,2'-indan]-1'-one) (spirendolol);

(7-[3-[[2-hydroxy-3-[(2-methylindol-4-yl)oxy]propyl]-amino]butyl]thiophylline) (teoprolol);

((±)-1-tert.butylamino-3-(thiochroman-8-yloxy)-2-propanol) (tertatolol);

((±)-1-tert.butylamino-3-(2,3-xylyloxy)-2-propanol HCl) (xibenolol);

(8-[3-(tert.butylamino)-2-hydroxypropoxy]-5-methyl-coumarin) (bucumolol);

(2-(3-(tert.butylamino)-2-hydroxy-propoxy)benzonitrile HCl) (bunitrolol);

((±)-2'-[3-(tert-butylamino)-2-hydroxypropoxy-5'-fluorobutyrophenone) (butofilolol);

(1-(carbazol-4-yloxy)-3-(isopropylamino)-2-propanol) (carazolol);

(5-(3-tert.butylamino-2-hydroxy)propoxy-3,4-dihydro-carbostyril HCl) (carteolol);

(1-(tert.butylamino)-3-(2,5-dichlorophenoxy)-2-propanol) (cloranolol);

(1-(inden-4(or 7)-yloxy)-3-(isopropylamino)-2-propanol HCl) (indenolol);

(1-isopropylamino-3-[(2-methylindol-4-yl)oxy]-2-propanol) (mepindolol);

(1-(4-acetoxy-2,3,5-trimethylphenoxy)-3-isopropylamino-propan-2-ol) (metipranolol);

(1-(isopropylamino)-3-(o-methoxyphenoxy)-3-[(1-methyl-ethyl)amino]-2-propanol) (moprolol);

((1-tert.butylamino)-3-[(5,6,7,8-tetrahydro-cis-6,7-dihydroxy-1-naphthyl)oxy]-2-propanol) (nadolol);

((S)-1-(2-cyclopentylphenoxy)-3-[(1,1-dimethylethyl)-amino]-2-propanol sulfate (2:1)) (penbutolol);

(4'-[1-hydroxy-2-(amino)ethyl]methanesulfonanilide) (sotalol);

(2-methyl-3-[4-(2-hydroxy-3-tert.butylaminopropoxy)-phenyl]-7-methoxy-isoquinolin-1-(2H)-one);

(1-(4-(2-(4-fluorophenyloxy)ethoxy)phenoxy)-3-isopropylamino-2-propanol HCl);

((-)-p-[3-[(3,4-dimethoxyphenethyl)amino]-2-hydroxy-propoxy]-ß-methylcinnamonitrile) (pacrinolol);

(($\pm$)-2-(3'-tert.butylamino-2'-hydroxypropylthio)-4-(5'-carbamoyl-2'-thienyl)thiazole HCl) (arotinolol);

(($\pm$)-1-[p-[2-(cyclopropylmethoxy)ethoxy]phenoxy]-3-(isopropylamino)-2-propanol) (cicloprolol);

(($\pm$)-1-[(3-chloro-2-methylindol-4-yl)oxy]-3-[(2-phenoxyethyl)amino]-2-propanol) (indopanolol);

(($\pm$)-6-[[2-[[3-(p-butoxyphenoxy)-2-hydroxypropyl]-amino]ethyl]amino]-1,3-dimethyluracil) (pirepolol);

(4-(cyclohexylamino)-1-(1-naphtholenyloxy)-2-butanol);

(1-phenyl-3-[2-[3-(2-cyanophenoxy)-2-hydroxypropyl]-aminoethyl]hydantoin HCl);

(3,4-dihydro-8-(2-hydroxy-3-isopropylaminopropoxy)-3-nitroxy-2H-1-benzopyran) (nipradolol);

<u>α and ß-Adrenergic Blocking Agents</u>:

(($\pm$)-1-tert-butylamino)-3-[o-[2-(3-methyl-5-isoxazolyl)vinyl]phenoxy]-2-propanol) (isoxaprolol);

(1-isopropylamino-3-(4-(2-nitroxyethoxy)phenoxy)-2-propanol HCl);

(4-hydroxy-α-[[3-(4-methoxyphenyl)-1-methylpropyl]-aminomethyl]-3-(methylsulfinyl)-benzmethanol HCl) (sulfinalol);

(5-[1-hydroxy-2-[[2-(o-methoxyphenoxy)ethyl]amino]-ethyl]-2-methylbenzenesulfonamide HCl);

(5-[1-hydroxy-2-[(1-methyl-3-phenylpropyl)amino]ethyl]-salicylamide HCl) (labetalol);

(1-((3-chloro-2-methyl-1H-indol-4-yl)oxy)-3-((2-phenoxyethyl)amino)-2-propanol-hydrogenmalonate) (ifendolol);

(4-(2-hydroxy-3-[(1-methyl-3-phenylpropyl)amino]-propoxy)benzeneacetamide);

(1-[3-[[3-(1-naphthoxy)-2-hydroxypropyl]-amino]-3,3-dimethyl-propyl]-2-benzimidazolinone);

(3-(1-(2-hydroxy-2-(4-chlorophenylethyl)-4-piperidyl)-3,4-dihydroxy)quinoxolin-2(1H)-one);

<u>CNS-Acting Agents</u>: clonidine; methyldopa;

<u>Adrenergic Neuron Blocking Agents</u>: guanethidine; reserpine and other rauwolfia alkaloids such as rescinnamine;

<u>Vasodilators</u>: diazoxide; hydralazine; minoxidil;

<u>Angiotensin I Converting Enzyme Inhibitors</u>:

1-(3-mercapto-2-methyl-1-oxopropyl)-L-proline (captopril);

(1-(4-ethoxycarbonyl-2,4(R,R)-dimethylbutanoyl)-indoline-2(S)-carboxylic acid);

(2-[2-[[1-(ethoxycarbonyl)-3-phenyl-propyl]amino]-1-oxopropyl]-1,2,3,4-tetrahydro-3-isoquinoline carboxylic acid);

((S)-1-[2-[[1-(ethoxycarbonyl)-3-phenylpropyl]amino]-1-oxopropyl]octahydro-1H-indole-2-carboxylic acid HCl);

(N-cyclopentyl-N-(3-(2,2-dimethyl-1-oxopropyl)thiol-2-methyl-1-oxopropyl)glycine) (pivalopril);

((2R,4R)-2-(2-hydroxyphenyl)-3-(3-mercaptopropionyl)-4-thiazolidinecarboxylic acid);

(1-(N-[1(S)-ethoxycarbonyl-3-phenylpropyl]-(S)-alanyl)-cis,syn-octahydroindol-2(S)-carboxylic acid HCl);

((-)-(S)-1-[(S)-3-mercapto-2-methyl-1-oxopropyl]-indoline-2-carboxylic acid);

([1(S),4S]-1-[3-(benzoylthio)-2-methyl-1-oxopropyl]-4-phenylthio-L-proline;

(3-([1-ethoxycarbonyl-3-phenyl-(1S)-propyl]amino)-2,3,4,5-tetrahydro-2-oxo-1-(3S)-benzazepine-1-acetic acid HCl);

(N-(2-benzyl-3-mercaptopropanoyl)-S-ethyl-L-cysteine) and the S-methyl analogue;

(N-(1(S)-ethoxycarbonyl-3-phenylpropyl)-L-alanyl-L-proline maleate) (enalapril);

N-[1-(S)-carboxy-3-phenylpropyl]-L-alanyl-1-proline;

$N^2$-[1-(S)-carboxy-3-phenylpropyl]-L-lysyl-L-proline (lysinopril);

2-[N-[(S)-1-ethoxycarbonyl-3-phenylpropyl]-L-alanyl-(1S,3S,5S)-2-azabicyclo[3.3.0]octane-3-carboxylic acid;

[1(S),4S]-1-(3-mercapto-2-methyl-1-oxopropyl)-4-phenylthio-L-proline S-benzoyl calcium salt (zofenopril);

[1-(+/-)4S]-4-cyclohexyl-1-[[2-methyl-1-[1-(1-(oxy-propoxy)propoxy](4-phenylbutyl)phosphinyl]acetyl]-L-proline sodium salt (fosfopril).

Calcium Channel Blockers: nifedepine; nitrendipine, verapamil; diltiazam.

Other Antihypertensive Agents: aminophylline; cryptenamine acetates and tannates; deserpidine; meremethoxylline procaine; pargyline; trimethaphan camsylate;
and the like, as well as admixtures and combinations thereof.

Typically, the individual daily dosages for these combinations can range from about one-fifth of the minimally recommended clinical dosages to the maximum recommended levels for the entities when they are given singly. Coadministration is most readily accomplished by combining the active ingredients into a suitable unit dosage form containing the proper dosages of each. Other methods of coadministration are, of course, possible.

The novel compounds of the present invention possess an excellent degree of activity in treating hypertension and congestive heart failure.

For these purposes the compounds of the present invention may be administered orally, parenterally, by inhalation spray, or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, dogs, cats, etc., the compounds of the invention are effective in the treatment of humans.

The pharmaceutical compositions may be in the form of a sterile injectable preparation, for example as a sterile injectable aqueous or oleagenous suspension. This suspension may be formulated according to the known art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The peptides of this invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

Dosage levels of the order of 0.1 to 4.0 grams per day parenterally are useful in the treatment of the above indicated conditions. Oral doses are 3-10 times higher. For example, renin-associated hypertension and hyperaldosteronism are

effectively treated parenterally by the administration of from 1.0 to 50 milligrams of the compound per kilogram of body weight per day.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

Thus, in accordance with the present invention there is further provided a pharmaceutical composition for treating hypertension and congestive heart failure, comprising a pharmaceutical carrier and a therapeutically effective amount of a compound of the formula I.

Also, in accordance with the present invention there is still further provided a method of treating hypertension and congestive heart failure, comprising administering to a patient in need of such treatment, a therapeutically effective amount of a compound of the formula I.

The renin inhibitory compounds of the present invention may also be utilized in diagnostic methods for the purpose of establishing the significance of renin as a causative or contributory factor in hypertension or congestive heart failure in

a particular patient. For this purpose the present compounds may be administered in a single dose of from 0.1 to 10 mg per kg of body weight.

Both in vivo and in vitro methods may be employed. In the in vivo method, a novel peptide of the present invention is administered to a patient, preferably by intravenous injection, although other routes of parenteral administration are also suitable, at a hypotensive dosage level and as a single dose, and there may result a transitory fall in blood pressure. This fall in blood pressure, if it occurs, indicates supranormal plasma renin levels.

Some of the compounds of formula I also have angiotensin converting enzyme (ACE) inhibitor activity. This activity augments renin inhibition in lowering blood pressure and in treating congestive heart failure. Treatment dosage and mode for this utility is generally the same as set out earlier.

The preparation of the formula I compounds is illustrated in some of the examples below and in general proceeds as follows:

a) Coupling of an amino-protected form of E to G, followed by b) amino protecting group removal from E-G and c) coupling A to the resulting amino group of E-G.

The phosphorous-containing component E, as well as the component A may contain functionality which requires protection during the coupling reactions. Protecting groups, among those well-known in peptide synthesis, are chosen so as to be compatible with the coupling steps, yet easily removable afterwards. Among those utilized for amino

group protection are the t-butoxycarbonyl (BOC), benzyloxycarbonyl (CBZ), 9-fluorenylmethyloxycarbonyl (FMOC), and benzyl groups. Carboxylic acids are protected as the methyl, ethyl, benzyl, or t-butyl esters. Phosphonic and phosphinic acids are protected as the methyl or ethyl esters. The coupling procedures referred to above include those brought about by use of dicyclohexylcarbodiimide/ 1-hydroxybenzotriazole and of disuccinimido oxallate (K. Takeda et al., Tetrahedron Lett., 24, 4451-54 (1983)). In many cases, both carboxylic and phosphonic (or phosphinic esters) may be hydrolyzed along with amino protecting groups as the last step in the synthesis. In these cases, treatment of the phosphorous-containing peptide analog with 30% hydrobromic acid in acetic acid, with 6N hydrochloric acid, or with aqueous sodium hydroxide, followed by purification of the resulting deprotected material by ion-exchange chromatography or reverse-phase HPLC, provides the desired product. In instances where A-E-G possesses a carboxyl-terminal amide function ($R_3$=$NH_2$, $NHR^3_a$, or $NR^3_aR^3_a$), the fully coupled material A-E may be treated with 1 equivalent of lithium hydroxide (0.1 N) to hydrolyze selectively a carboxylic ester function in E. Standard coupling procedures may then be used to couple the resulting free carboxylic acid to an appropriate amine. This is followed by removal of the remaining protecting groups as described above. Alternatively the amide formation may be carried out prior to coupling of the component E-G to the A unit.

Preparation of the phosphorous-containing components E are carried out as illustrated in the examples to follow.

The 1-aminoalkylphosphonous acids used as starting materials in the examples below are prepared as illustrated in the examples and can be resolved to give optically active materials by the method of Baylis et al. (J. Chem. Soc. Perkin Trans 1, 2845-53 (1984)). Compounds derived from both the optically active and racemic materials are claimed in the present invention.

The 1-aminoalkylphosphonic acids used in the examples below are prepared as described and can be resolved to give the optically active materials by the procedure of Kafarski et al., Can. J. Chem., 60, 3081-84 (1982), or can be prepared in optically active form by the method of Huber et al., Tetrahedron Lett., 3049-3052 (1979). Compounds derived from both the optically active and racemic materials are claimed in the present invention.

When the amino group of the unit E which is to be coupled to A bears an N-methyl group ($R^6$ = $-CH_3$), procedures well known in peptide synthesis are used to couple A to E or E-G. In general, the mixed anhydride procedure (with pivaloyl chloride and N-methylmorpholine) is used, as illustrated by R. M. Wenger, Helv. Chem. Acta., 1984, 67, 502-525. This procedure is also utilized in formation of E-G when G is 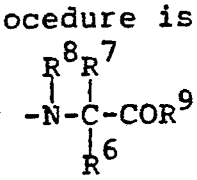 and $R^8$ is other than hydrogen and in the preparation of G when $R^9$ is 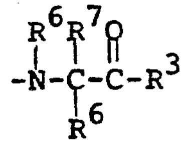 and $R^6$ is methyl.

The following schemes and examples illustrate the preparation of compounds of formula I. In these schemes and examples, the following abbreviations are used: DPPA = diphenylphosphorylazide; DCC = dicyclohexylcarbodiimide; HOBT = 1-hydroxybenzotriazole hydrate; TFA = trifluoroacetic acid; CBZ = carbobenzoxy; BOC = t-butoxycarbonyl; DNP = 2,4-dinitrophenyl; FMOC = 9-fluorenylmethyloxycarbonyl; Bz = benzyl; HOAc = acetic acid; TMS-Cl = trimethylsilyl chloride; AIBN = azobis isobutyronitrile.

It will be understood that the following schemes outline representative examples of the preparation of peptides of formula I and that similar peptides possessing alternative substituents can equally well be prepared by the routes outlined.

The component E may be, for example, a phosphinic acid of the formula II.

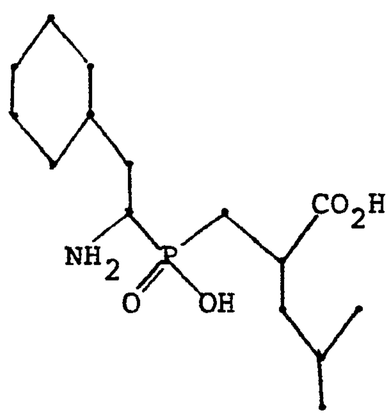

II

Compound VIII containing II may be prepared as outlined in the scheme below:

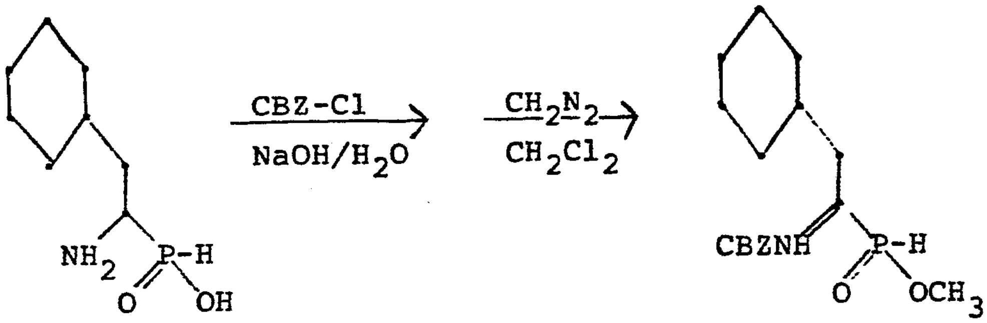
CBZ-Cl
NaOH/H2O
CH2N2
CH2Cl2

III IV

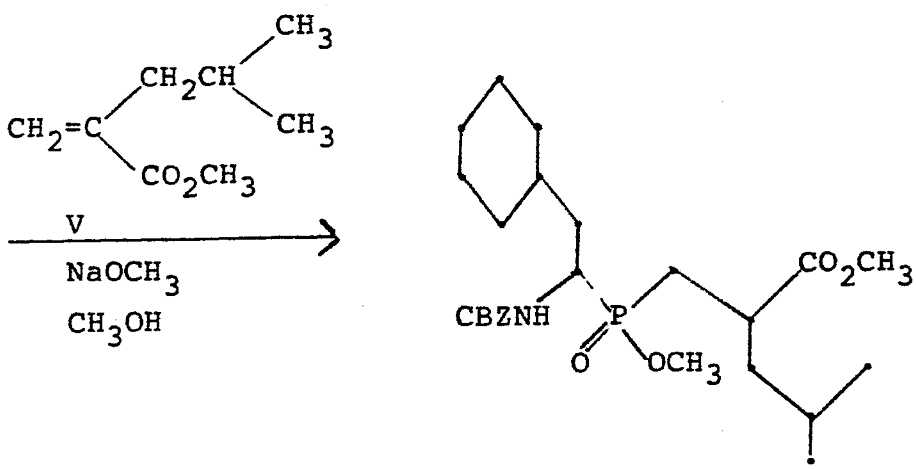
V
NaOCH3
CH3OH

VI

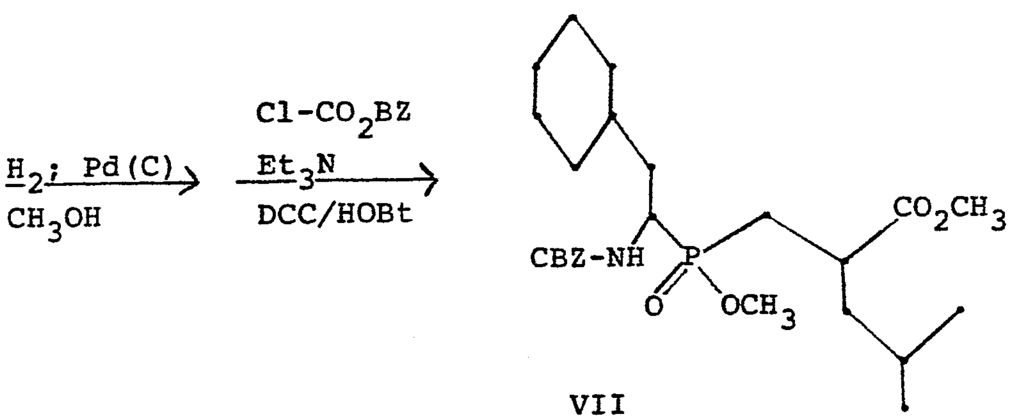
H2; Pd(C)
CH3OH
Cl-CO2BZ
Et3N
DCC/HOBt

VII

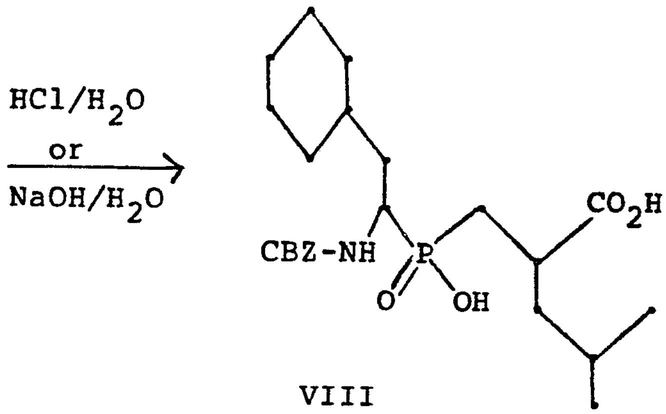


VIII

An alternative procedure for preparation of phosphinate VI is illustrated in the scheme below:

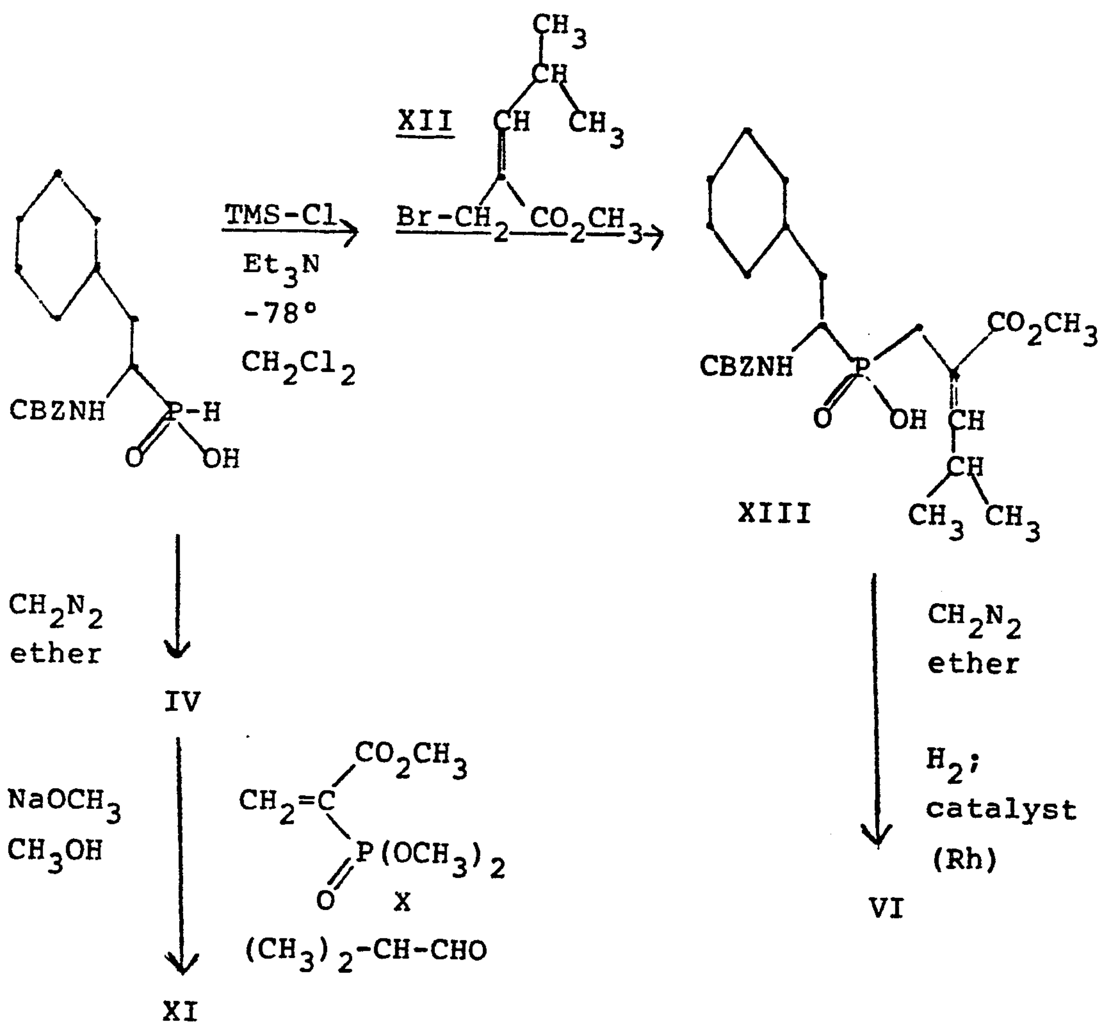


XII

XIII

IV

X

XI

VI

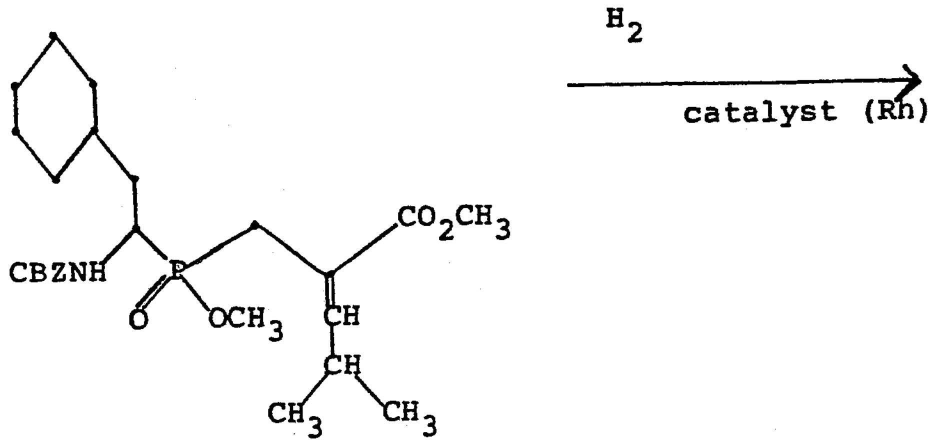


VI

XI

Component E may be for example a phosphinic acid of formula XIV:

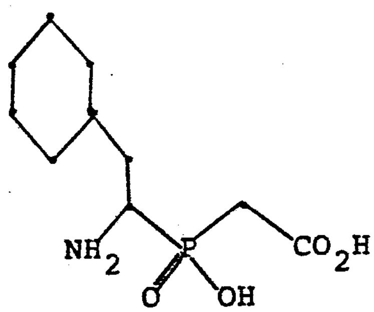

XIV

Preparation of protected forms XVII and XXII of XIV is illustrated in the schemes below:

Route A:

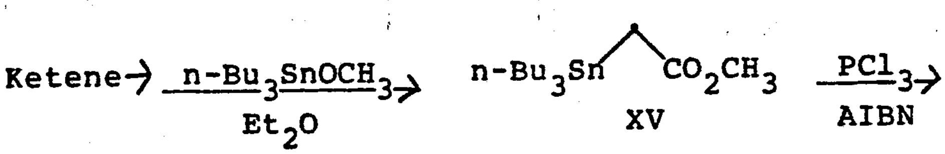


XV

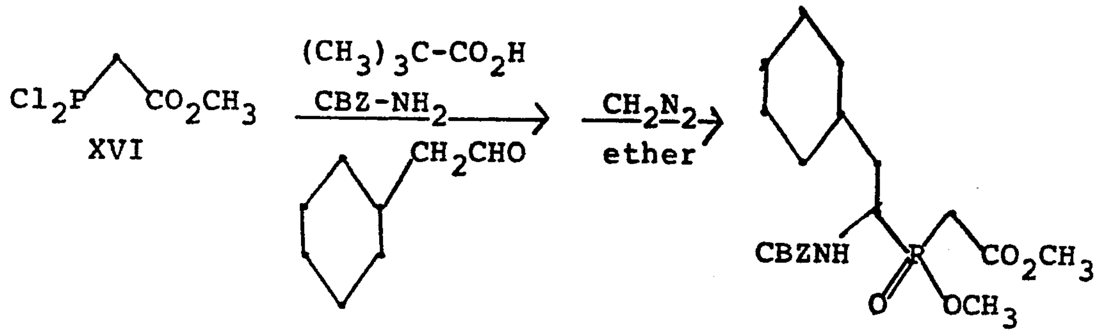


XVI XVII

Route B:

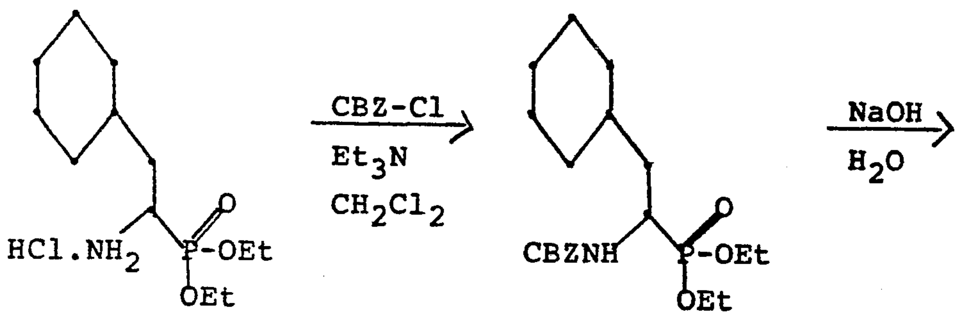


XVIII XIX

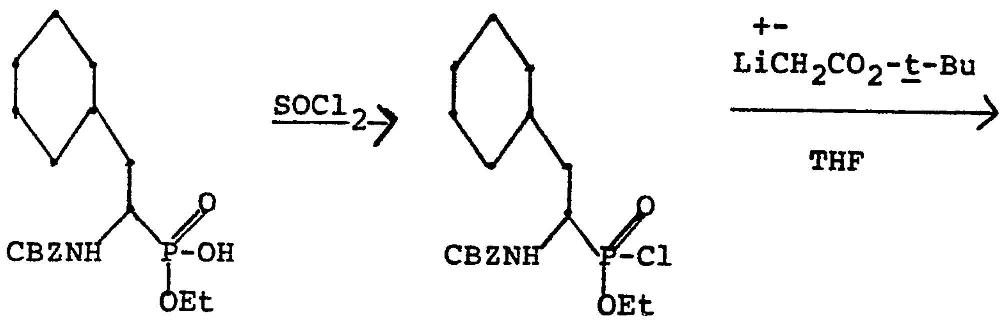


XX XXI

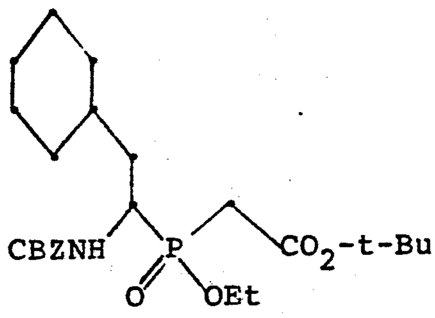

XXII

Route C:

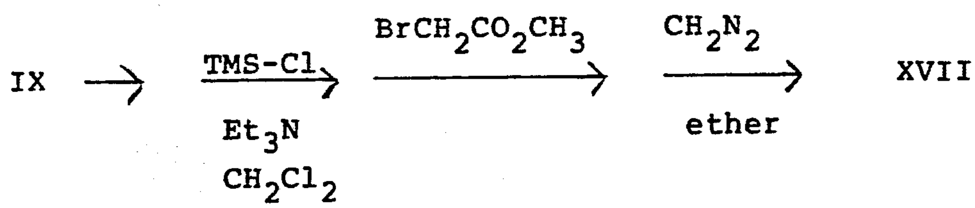


Phosphinic esters XVII and XXII may be coupled to an A unit as illustrated above in preparation of peptide VIII.

The component E may be a phosphinic ester of the formula XXIII:

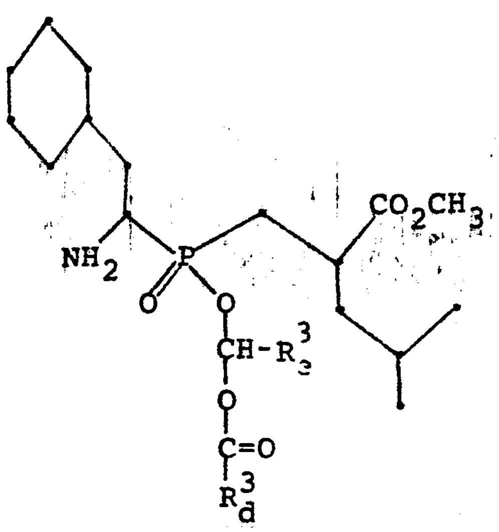

XXIII

Compound XXVII containing such a component E may be prepared as outlined in the following scheme:

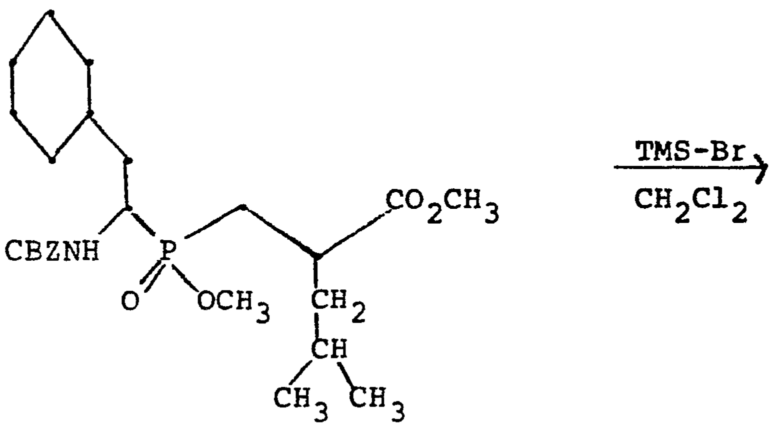


VI

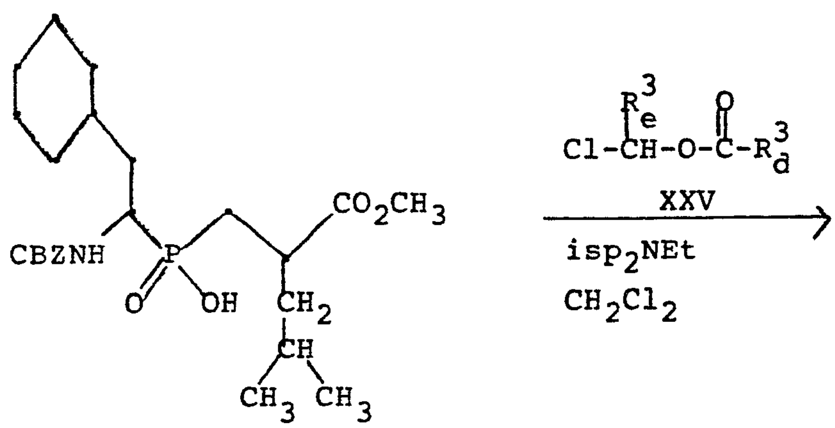


XXIV

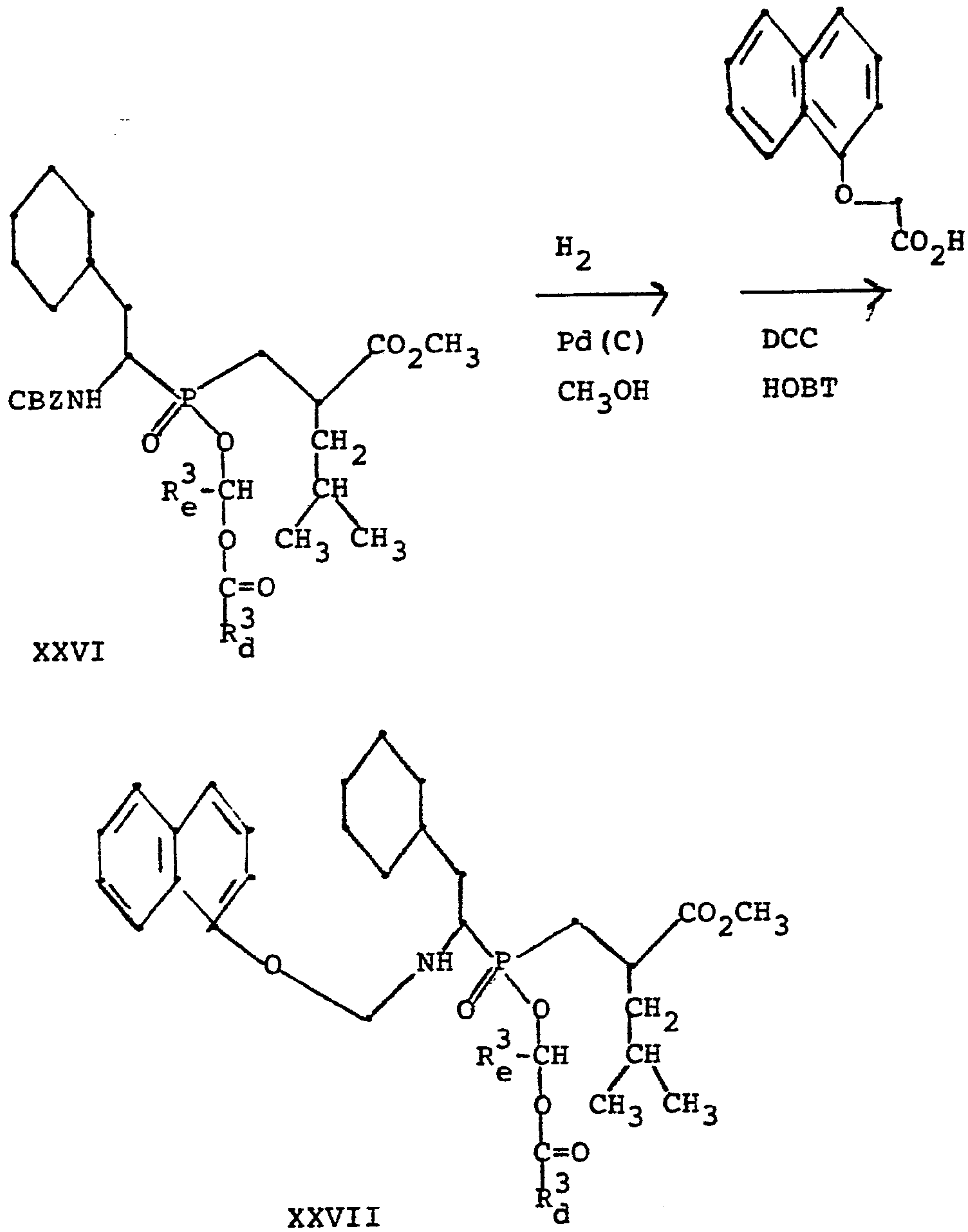


XXVI

XXVII

Components XXV may be prepared as illustrated below:

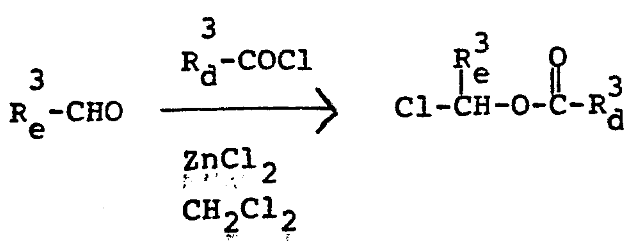

Melting points were recorded on a Thomas-Hoover melting point apparatus and are uncorrected as are all boiling points. $^{1}H$ NMR spectra were taken on a Varian XL-300 FT spectrometer. Chemical shifts are reported in ppm downfield from tetramethylsilane as internal standard. IR spectra were recorded on a Perkin-Elmer Model 297 spectrometer. Optical rotations were measured with a Perkin Elmer 141 automatic polarimeter in the solvents indicated. Mass spectra (MS) were taken on a Varian 731 spectrometer at 70 eV. Those marked FAB were taken by using the fast atom bombardment method.

Examples 1-8, 17-23, 25-37, 39-40, 56-62 and 70 illustrate or pertain to the present invention. All temperatures are in °C unless otherwise indicated.

EXAMPLE 1

Cyclohexylacetaldehyde

A suspension of 100 g (0.46 moles) of pyridinium chlorochromate and 100 g of celite in 800 ml of methylene chloride was stirred vigorously while 38 g (0.3 moles) of 2-cyclohexylethanol in 200 ml of methylene chloride was added all at once. The reaction turned dark immediately and became mildly exothermic. After 1 hour, 1000 ml of ether was added and the reaction mixture was filtered through a bed of silica gel (ca. 250 g) on a fritted glass disk. The pad was rinsed with an additional liter of ether. The combined filtrates were reduced in volume to approximately 200 ml and the solution was washed with 2 x 40 ml of 6N HCl, 1 x 50 ml of saturated sodium bicarbonate, and 1 x 50 ml of saturated NaCl

solution. The organic layer was dried over magnesium sulfate, filtered and evaporated in vacuo to give a light green oil. The residue was distilled in vacuo to afford 21 g (56%) of a colorless oil (bp 74-76°C at 23 mm of Hg). NMR ($CDCl_3$) (60 MHz): 0.8-2.1 (m, 11H); 2.2-2.4 (m, 2H); 9.6 (t, J=2 Hz, 1H) ppm.

EXAMPLE 2

1-Amino-2-cyclohexylethylphosphinic acid

A stirred slurry of 26.00 g (0.118 moles) of aminodiphenylmethane-HCl in 100 ml of absolute ethanol was treated with 15.50 g (0.123 moles) of cyclohexylacetaldehyde, immediately followed by 12.8 ml (0.123 moles) of hypophosphorous acid (50% aqueous). The reaction mixture was heated in an oil bath held at 100°C. As the reaction approached reflux it became homogeneous, then heterogeneous again after approximately 5 minutes (white precipitate). After 45 minutes at reflux, an additional 100 ml of ethanol was added to the very thick slurry. Reflux was continued for an additional 3 hours 15 minutes. At this time the reaction mixture was cooled to 0°C in an ice bath, then the solid filtered off. The white solid was washed with 50 ml of ice-cold ethanol and air dried.

The white solid was added to 150 ml of glacial acetic acid and 150 ml of 48% aqueous HBr added. Dissolution occurred over a period of 5 minutes, and the reaction turned a light yellow color. After another 10 minutes a white solid precipitated out of solution. Stirring at room temperature was continued for a total of 2 hours. The flask was then immersed in an oil bath preheated

to 115°C. After 1 hour the reaction was almost homogeneous. After a total of 3 hours at 115°C, the reaction mixture was cooled to 0°C in an ice bath. The solution was washed with 1 x 200 ml and 2 x 100 ml of hexanes. The hexanes wash was discarded and the remaining aqueous acid solution was evaporated to dryness on a rotary evaporator. The resulting semi-crystalline foam was dissolved in 125 ml of absolute ethanol and cooled to 0°C in an ice bath. Propylene oxide (50 ml) was slowly added and a white precipitate was formed. The reaction mixture was allowed to warm to room temperature of its own accord while stirring. After a total of 18 hours, the slurry was cooled to 0°C again, and the solid filtered off. The solid was washed with 100 ml more of ice-cold ethanol and dried to afford 11.98 g (53% overall) of a white solid mp 220-221°C (turns orange and bubbles). NMR ($D_2O$) (60 MHz): 0.8-2.1 (m, 14H); 3.3 (m, 2H); 7.0 (d, J=527 Hz, 1H) ppm.

## EXAMPLE 3

### Methyl N-CBZ-1-Amino-2-cyclohexylethylphosphinate

A solution of 7.00 g (0.037 moles) of 1-amino-2-cyclohexylethylphosphonous acid in 105 ml of dioxane and 40 ml of 1N NaOH was cooled to 0°C in an ice bath and stirred vigorously while 10.50 ml (12.53 g; 0.073 moles) of benzyl chloroformate and 80 ml of 1N NaOH were added rapidly and simultaneously over a period of approximately 1 minute. The pH was adjusted to the 8-9 range using 1N NaOH (hydrion paper) added in small increments. The reaction mixture was allowed to come to room temperature, and vigorous stirring was continued for 48 hours. The

dioxane was removed *in vacuo* and the aqueous washed with 100 ml of ether. The ether layer was discarded and the aqueous solution acidified using 1N $KHSO_4$ to approximately pH = 1-2 (hydrion paper). The solution was extracted with 5 x 100 ml of ethyl acetate. The combined ethyl acetate layers were dried over anhydrous $Na_2SO_4$, filtered, and evaporated *in vacuo* to afford N-CBZ-1-amino-2-cyclohexylethylphosphonous acid as a white solid.

The solid was redissolved in 200 ml of ethyl acetate and 150 ml of ethereal diazomethane was added all at once. The reaction was stirred at room temperature for 1 hour, at which time the volatiles were removed completely *in vacuo* to give a viscous oil. This material was crystallized using 200 ml of 1:1 ether:petroleum ether to afford 3.45 g of product. The mother liquors obtained after evaporation of the filtrate were chromatographed on silica gel using 18:1:1 methylene chloride:acetone:methanol to give an additional 5.69 g of product. Total product = 9.14 g (73%). NMR ($CDCl_3$) (60 MHz): 0.8-2.2 (m, 14H); 3.6, 3.8 (s, 3H); 3.8-4.6 (br, 1H); 5.2 (s, 2H); 6.9 (d, J=542 Hz, 1H); 7.2 (s, 5H) ppm.

## EXAMPLE 4

### Methyl N-BOC-1-amino-2-cyclohexylethylphosphinate

A solution of 0.955 g (0.005 moles) of 1-amino-2-cyclohexylethylphosphonous acid in 10 ml of dioxane and 10 ml of 0.5N NaOH was cooled to 0°C in an ice bath. The reaction mixture was vigorously stirred while 1.26 ml (1.20 g; 0.0055 moles) of di-tert-butyldicarbonate was added all at once. The reaction mixture was allowed to come to room

temperature. After a total reaction time of 17 hours, the dioxane was removed in vacuo and the aqueous was acidified with 1N $KHSO_4$. The mixture was extracted with 3 x 50 ml of ethyl acetate. The combined ethyl acetate solution was dried (anhydrous $Na_2SO_4$) and evaporated in vacuo to a slightly cloudy oil.

The oil was redissolved in 50 ml of ethyl acetate and treated with 50 ml of ethereal diazomethane solution. The reaction mixture was stirred at room temperature for 2 hours, then the volatiles were removed completely in vacuo to give a thick oil. The crude product was chromatographed on silica gel using 18:1:1 methylene chloride:acetone:methanol as the eluant to give 1.17 g (100%) of product as a very thick oil. NMR ($CDCl_3$) (60 MHz): 0.0-2.2 (m containing 9H s at 1.5, 23H (total)); 3.7, 3.9 (s, 3H); 5.2-6.0 (m, 1H); 6.9 (d, J=552 Hz, 1H) ppm.

## EXAMPLE 5

Methyl (N-CBZ-1-amino-2-cyclohexylethyl) 2-carbomethoxy-4-methylpentylphosphinate

A solution of 2.75 g (0.008 moles) of methyl 1-CBZ-amino-2-cyclohexylethylphosphinate in 25 ml of absolute methanol was cooled to 0°C in an ice bath and 4.60 ml of 2M NaOMe in methanol (0.009 moles) was added via syringe. The reaction mixture was stirred at 0°C for 10 minutes, at which time 1.21 g (0.009 moles) of methyl 2-(2-methylpropyl)acrylate was added all at once. The reaction was allowed to proceed at 0°C for 30 minutes, then the ice bath was removed and the reaction was allowed to proceed at room temperature for 18 hours. The methanol was removed in vacuo

and the residue treated with 100 ml of 1N HCl. The aqueous was extracted with 3 x 50 ml of ethyl acetate. The combined ethyl acetate was dried over anhydrous $MgSO_4$, filtered, and evaporated *in vacuo* to give a colorless oil. The crude product was chromatographed on silica gel using 18:1:1 methylene chloride:acetone:methanol as the eluant to afford 1.91 g (49%) of the product as a white solid. NMR ($CDCl_3$) (60 MHz): 0.8-2.2 (m, 14H); 3.6, 3.8 (s, 3H); 5.1 (s, 2H); 7.0 (d, J=535 Hz, 1H); 7.3 (s, 5H) ppm.

## EXAMPLE 6

Methyl (N-BOC-1-amino-2-cyclohexylethyl) 2-carbomethoxy-4-methylpentylphosphinate

A solution of 4.85 g (0.021 moles) of the ester from Example 4 in 35 ml of absolute methanol was cooled to 0°C, whereupon 11.45 ml of 2N NaOMe in methanol (0.023 moles) was added *via* syringe. The reaction was stirred for 10 minutes, at which time 3.10 g (0.022 moles) of methyl 2-(2-methylpropyl)-acrylate was added all at once. Stirring was continued at 0°C for 30 minutes, then the ice bath was removed and stirring was continued at room temperature for 19 hours. At that time the methanol was removed *in vacuo* and the residue treated with 200 ml of 1N HCl. The aqueous was extracted with 3 x 50 ml of ethyl acetate. The combined ethyl acetate washes were dried ($MgSO_4$), filtered and the volatiles evaporated *in vacuo* to give an oil. The crude product was purified by silica gel chromatography using ethyl acetate as an eluant to afford 4.77 g (61%) of the product as a very viscous oil.

NMR ($CDCl_3$) (60 MHz): 0.9 (m, 6H); 0.8-2.2 (m, containing 9H s at 1.5, 29H total); 3.6, 3.8 (s, 3H); 3.7 (s, 3H) ppm.

## EXAMPLE 7

Methyl (1-amino-2-cyclohexylethyl) 2-carbomethoxy-4-methylpentylphosphinate

A mixture of 1.86 g (0.004 moles) of the ester product of Example 5 and 0.95 g of 10% Pd on carbon in 30 ml of absolute methanol was hydrogenated on a Parr type apparatus at 40 psig of hydrogen for 20 hours. The reaction mixture was filtered through a small pad of celite and the pad washed well with methanol. The filtrate was evaporated completely *in vacuo* to afford the pure free amine (1.34 g; 100%) as a viscous oil. NMR ($CDCl_3$) (300 MHz) 0.8-1.0 (m, 6H); 0.8-3.0 (m, 20H); 3.7-4.0 (series of s, total 6H); 8.1 (very br s, 2H) ppm.

## EXAMPLE 8

Methyl (1-amino-2-cyclohexylethyl) 2-carbomethoxy-4-methylpentylphosphinate hydrochloride

A solution of 1.13 g (0.003 moles) of the product of Example 6 in 20 ml of absolute methanol was treated with 12 ml of HCl/methanol (144 g of HCl in 400 ml methanol). The reaction mixture was stirred at room temperature for 4.5 hours. Analysis by thin layer chromatography indicated that no more starting material remained. The volatiles were removed *in vacuo* and replaced several times with more methanol and re-evaporated. After vacuum drying, this afforded 1.07 g (100%) of the product as a

glassy foam. NMR ($CDCl_3$) (60 MHz): 0.9 (m, 6H); 0.8-3.0 (m, 20H); 3.5-4.1 (m, 6H); 8.6 (very br s, 3H) ppm.

## EXAMPLE 9

**Methyl [N-(N-BOC-$N^{im}$-DNP-histidyl)-1-amino-2-cyclohexylethyl] 2-carbomethoxy-4-methylpentylphosphinate**

A mixture of 0.694 g (0.002 moles) of the product of Example 7, 0.88 g (0.002 moles) of N-BOC-$N^{im}$-(2,4-dinitrophenyl)histidine, and 0.297 g (0.0022 moles) of HOBT in 20 ml of dry methylene chloride was stirred at room temperature until the reaction mixture was homogeneous (ca. 1 hour). The reaction mixture was cooled to 0°C in an ice bath and 0.453 g (0.0022 moles) of DCC was added all at once. The reaction was allowed to come to room temperature of its own accord, and slowly became heterogeneous. After stirring at room temperature for 20 hours, the mixture was diluted with 100 ml of ether and filtered through celite. The filtrate was washed with 2 x 20 ml of saturated $NaHCO_3$, dried over anhydrous $Na_2SO_4$, filtered and the volatiles removed *in vacuo* to give a crude yellow foam. The crude material was chromatographed on silica gel using 18:1:1 methylene chloride:acetone:methanol as the eluant to give 0.949 g (63%) of the product as a light yellow glassy foam. NMR ($CDCl_3$) (300 MHz): 0.8-1.0 (m, 6H): 1.0-2.2 (m containing a 9H s at 1.5, total=28H); 2.8 (br s, 1H); 3.1 (m, 2H); 3.6-3.8 (series of s, total=6H); 4.4 (m, 2H); 5,9-6.2 (m, 1H); 6.8-7.4 (m, 2H); 7.5-7.9 (m, 2); 9.6 (dd, 1H); 8.8 (d, 1H) ppm.

EXAMPLE 10

Methyl [N-($N^{im}$-DNP-histidyl)-1-amino-2-cyclohexyl] 2-carbomethoxy-4-methylpentylphosphinate hydrochloride

A solution of 0.949 g (0.0013 moles) of the product of Example 9 in 10 ml of absolute methanol was treated with 15 ml of HCl in methanol (144 g HCl/400 ml methanol). The reaction mixture was stirred at room temperature for 1 hour. The volatiles were removed completely in vacuo to afford 0.795 g (90%) of the crude product as a brown foam. NMR ($CDCl_3$) (300 MHz): 0.8-1.0 (m, 6H); 0.9-2.4 (m, 20H); 2.9 (br s, 2H); 3.2 (m, 1H); 3.7 (br s, 6H); 7.0 (m, 1H); 7.5-7.9 (m, 2H); 8.6 (m, 1H); 8.8 (m, 1H) ppm.

EXAMPLE 11

N-CBZ-2-Amino-3-(1-naphthyl)propionic acid

A solution of 0.700 g (0.003 moles) of 2-amino-3-(1-naphthyl)propionic acid in 10 ml of dioxane and 3.2 ml of 1N NaOH was cooled to 0°C in an ice bath and 6.40 ml of 1N NaOH and 0.923 ml (1.10 g; 0.0064 moles) of benzyl chloroformate were added simultaneously and rapidly via syringe. The reaction mixture was stirred vigorously and the pH was adjusted to the 8-9 range by adding 1N NaOH dropwise. The reaction was allowed to come to room temperature of its own accord while stirring vigorously. After 24 hours at room temperature, the dioxane was removed in vacuo and the aqueous washed with 2 x 10 ml of ether. The ether layers were discarded and the aqueous layer was acidified to pH=1-2 (hydrion paper) using 1N $KHSO_4$. The aqueous solution was extracted with 2 x 50 ml of ethyl acetate. The combined ethyl

acetate washings were dried over anhydrous $MgSO_4$, filtered, and the volatiles removed in vacuo to give a clear oil. This was triturated with 5 x 5 ml portions of petroleum ether and dried in vacuo to afford 1.09 g (97%) of the product. NMR ($CDCl_3$) (300 MHz): 3.4 (dd, 1H); 3.8 (dd, 1H); 4.8 (dd, 1H); 5.0 (s, 2H); 5.3 (d, 1H); 7.1-7.6 (m, 9H); 7.8 (d, 1H); 7.9 (d, 1H); 8.1 (d, 1H) ppm.

EXAMPLE 12

N-BOC-2-Amino-3-(1'-naphthyl)propionic acid

A solution of 0.700 g (0.003 moles) of 2-amino-3-(1'-naphthyl)propionic acid in 7 ml of dioxane and 13 ml of 0.5 N NaOH was cooled to 0°C in an ice bath and stirred vigorously while 0.82 ml (0.773 g; 0.0036 moles) of di-tert-butyl dicarbonate was added in one portion. The ice bath was removed and the heterogeneous reaction mixture was stirred vigorously at room temperature. After several hours, the reaction mixture became homogeneous. Vigorous stirring was continued for ca. 24 hours. The dioxane was removed in vacuo and the residue acidified to pH=1-2 with 1N $KHSO_4$. The aqueous was extracted with 2 x 50 ml of ethyl acetate. The combined organic extracts were dried over anhydrous $MgSO_4$, filtered, and the volatiles evaporated in vacuo to give a colorless oil. The oil was triturated 10 ml of petroleum ether to give 0.97 g (94%) of the product as a white powder (crystallization occurs very slowly). NMR ($CDCl_3$) (300 MHz): 0.7 (s, 9H); 3.2 (dd, 1H); 3.9 (dd, 1H); 4.7 (m, 1H); 7.3-7.6 (m, 5H); 7.8 (d, 1H); 7.9 (d, 1H); 8.2 (d, 1H) ppm.

## EXAMPLE 13

Methyl [N-(N-(N-CBZ-2-amino-3-(1-naphthyl)propionyl)-$N^{im}$-DNP-histidyl)-1-amino-2-cyclohexylethyl] 2-carbomethoxy-4-methylpentylphosphinate

A mixture of 0.176 g (0.0005 moles) of N-CBZ-2-amino-3-(1'-naphthyl)-propionic acid and .156 g (0.00055 moles) of disuccinimidyl oxalate in 7 ml of dry acetonitrile was treated with 0.045 ml (0.00055 moles) of dry pyridine. The reaction mixture was stirred at room temperature under nitrogen atmosphere for 20 hours. The mixture became homogeneous during this period. At this time, a solution of 0.300 g (0.00043 moles) of the product of Example 10 in a mixture of 8 ml of dry acetonitrile and 0.28 ml (0.202 g) of triethylamine was added all at once. The reaction turned very dark and stirring at room temperature under nitrogen was continued for 20 hours. The volatiles were evaporated in vacuo and the residue added to 30 ml of ethyl acetate, which was washed with 2 x 10 ml of saturated $NaHCO_3$, dried over anhydrous $Na_2SO_4$, filtered, and the volatiles removed in vacuo. The residue was chromatographed on silica gel using 18:1:1 methylene chloride:acetone:methanol as the eluant to afford 0.348 g (86%) of the tripeptide as a brownish-yellow glassy foam. NMR ($CDCl_3$) (300 MHz): 0.7-0.9 (m, 6H); 0.9-2.3 (m, 20H); 2.8-3.5 (m, 3H); 3.5-3.8 (series of singlets, total=6H); 4.1-4.8 (m, 2H); 5.0 (s, 2H); 5.3-5.6 (m, 1H); 6.8-7.6 (m, 14H); 7.8 (t, 1H); 7.9 (t, 1H); 8.2 (d, 1H); 8.5 (br, t, 1H); 8.8 (s, 1H) ppm.

## EXAMPLE 14

Methyl [N-(N-(N-BOC-2-amino-3-(1-naphthyl)propionyl)-$N^{im}$-DNP-histidyl)-1-amino-2-cyclohexylethyl] 2-carbomethoxy-4-methylpentylphosphinate

A suspension of 0.159 g (0.0005 moles) of N-BOC-2-amino-3-(1-naphthyl)propionic acid and 0.156 g (0.00055 moles) of disuccinimidyl oxalate in 7 ml of dry acetonitrile was treated with 0.045 ml (0.044 g; 0.00055 moles) of dry pyridine. After approximately 5 hours, the reaction was homogeneous. The reaction mixture was stirred under nitrogen atmosphere for an additional 15 hours at room temperature. At this time, a solution of 0.300 g (0.00043 moles) of the product of Example 10 in 8 ml of dry acetonitrile containing 0.280 ml (0.202 g; 0.002 moles) of triethylamine was added all at once. The reaction mixture turned very dark, and stirring under nitrogen was continued for 20 hours. The volatiles were removed in vacuo and the residue taken up in 30 ml of ethyl acetate. The organic solution was washed with 2 x 10 ml of saturated $NaHCO_3$, dried over anhydrous $Na_2SO_4$, filtered, and the volatiles evaporated in vacuo. The residue was chromatographed on silica gel using 18:1:1 methylene chloride:acetone:methanol as the eluant to afford 0.348 g (86%) of the desired product as a yellow glassy foam. NMR ($CDCl_3$) (300 MHz): 0.8-1.0 (m, 6H); 0.9-2.3 (m containing 9H s at 1.4, total=29H); 2.8-3.5 (m, 4H); 3.6-3.8 (series of singlets, total=6H); 4.6 (m, 2H); 4.7 (m, 1H); 5.0 (m, 1H); 6.9-8.0 (m, 10H); 8.2 (d, 1H); 8.6 (dd, 1H); 8.8 (t, 1H) ppm.

EXAMPLE 15

Methyl [N-(N-(N-CBZ-2-amino-3-(1-naphthyl)propionyl)-histidyl-1-amino-2-cyclohexylethyl] 2-carbomethoxy-4-methylpentylphosphinate

A solution of 0.100 g (0.0001 moles) of the product of Example 13 in 4 ml of dry DMF was put in a Fisher-Porter tube and approximately 20 ml of ammonia was condensed into the tube also. The reaction turned dark purple. The tube was sealed and stirring was continued for approximately 3 hours. During this time the color went from purple to pink. The tube was opened and the ammonia was allowed to evaporate. The remaining solution was transferred to a flask and the volatiles were removed completely *in vacuo*. The residue was chromatographed on silica gel, giving 0.018 g (22%) of the desired product as a light yellow oil. NMR ($CDCl_3$) (300 MHz): 0.8-1.0 (m, 6H); 1.0-1.8 (m, 23H); 1.9-3.4 (m, 2H); 2.8-3.1 (m, 1H); 3.4 (m, 1H); 3.7-3.8 (series of singlets, total=6H); 4.6 (m, 1H); 5.1 (s, 1H); 5.2 (br s, 2H); 5.6 (m, 1H); 7.2-7.7 (m, 11H); 7.8 (d, 1H); 7.9 (d, 1H); 8.3 (br d, 1H) ppm.

EXAMPLE 16

[N-(N-(N-CBZ-2-amino-3-(1-naphthyl)propionyl)histidyl-1-amino-2-cyclohexylethyl] 2-carboxy-4-methylpentyl-phosphinic acid disodium salt

A solution of 0.018 g (0.000022 moles) of the product of Example 15 in 1 ml of ethanol was treated with 0.44 ml of 0.10N NaOH solution (0.000044 moles). The reaction mixture was stirred at room temperature for 16 hours. The volatiles were evaporated completely *in vacuo* and the residue

triturated with anhydrous ether (3 x 2 ml) to give 11 mg (61%) of the disodium salt as a very light yellow amorphous solid. NMR ($CD_3OD$) (300 MHz): 0.8-1.0 (m, 6H); 1.0-1.8 (m, 21H); 2.7 (br s, 1H); 3.0 (dd, 1H); 3.6-3.9 (m, 4H); 4.1 (br s, 1H); 4.2 (dd, 1H); 4.5 (dd, 1H); 5.0 (d, 2H); 7.1-7.6 (m, 11H); 7.8 (dd, 1H); 7.9 (d, 1H); 8.2 (d, 1H) ppm.

EXAMPLE 17

1,2,3,6-Tetrahydrophenyl vinyl ether

This compound was prepared in 67% yield using the method of Burgstahler (A. W. Burgstahler and I. C. Norton, J. Amer. Chem. Soc., 1961, 83, 198-206).

EXAMPLE 18

1,2,3,6-Tetrahydrophenylacetaldehyde

This compound was prepared in quantitative yield by the thermal rearrangement of 1,2,3,4-tetrahydrophenyl vinyl ether at 195°C according to the procedure of Burgstahler (A. W. Burgstahler and I. C. Norton, J. Amer. Chem. Soc., 1961, 83, 198-206).

EXAMPLE 19

1-Amino-2-(1,2,3,6-tetrahydrophenyl)ethylphosphinic acid

A mixture of 11.58 g (0.053 moles) of benzhydrylamine hydrochloride and 6.80 g (0.055 moles) of 1,2,3,6-tetrahydrophenylacetaldehyde in 42 ml of absolute ethanol was treated with 5.75 ml of 50% aqueous hypophosphorous acid. The reaction mixture was placed in an oil bath preheated to 100°C and stirred vigorously. The reaction became homogeneous after about 5 minutes, then heterogeneous

again immediately thereafter. Heating at 100°C was continued for a period of 4 hours. At this time, the reaction mixture was cooled to 0°C in an ice bath, and the solid filtered off and washed with 3 x 10 ml portions of ice-cold ethanol. The solid was dried *in vacuo* to afford 11.00 g of solid product as a fluffy white powder.

The crude powder was dissolved in 120 ml of trifluoroacetic acid, and the resulting solution was heated to 100°C in an oil bath. After approximately 5 minutes, the reaction had turned dark purple. Heating was continued for 1 hour. The reaction mixture was cooled to room temperature and the residue was partitioned between 250 ml of water and 100 ml of ether. The ether layer was separated and the aqueous washed with another 100 ml of ether. The ether was discarded and the aqueous solution evaporated completely *in vacuo*. The remaining white solid was treated with 10 x 50 ml of methanol, each time evaporating the methanol on a rotary evaporator. The white solid that remained was triturated with 25 ml of anhydrous ether to afford 3.77 g (36% overall) of the desired product. NMR ($D_2O$) (60 MHz): 1.0-2.4 (m, 9H); 3.2 (m, 1H); 5.4-5.8 (m, 2H); 6.9 (d, J=528Hz, 1H) ppm.

## EXAMPLE 20

Methyl N-CBZ-1-amino-2-(1,2,3,6-tetrahydrophenyl)-ethylphosphinate

A solution of 2.50 g (0.013 moles) of 1-amino-2-(1,2,3,6-tetrahydrophenyl)ethylphosphonous acid in 15 ml of 1N NaOH and 30 ml of dioxane that had been cooled to 0°C in an ice bath was stirred

BAD ORIGINAL

vigorously while 3.75 ml (4.45 g; 0.026 moles) of benzyl chloroformate and 30 ml of 1N NaOH were added rapidly and simultaneously. The pH of the solution was adjusted to 8-9 (hydrion paper) by the dropwise addition of additional 1N NaOH. The reaction mixture was allowed to warm to room temperature of its own accord, and the reaction was stirred vigorously for a total of 19 hours. The dioxane was removed in vacuo and the remaining aqueous was washed with 50 ml of ether. The ether was discarded and the aqueous was acidified with 1N $KHSO_4$ to approximately pH=1-2 (hydrion paper). The mixture was extracted with 3 x 100 ml of ethyl acetate. The combined ethyl acetate layers were dried over anhydrous $Na_2SO_4$, filtered, and the volatiles evaporated in vacuo to yield a viscous oil.

The oil was redissolved in 75 ml of ethyl acetate and 75 ml of diazomethane in ether was added all at once. The reaction mixture was stirred at room temperature for 2 hours, then the volatiles were evaporated completely in vacuo. The residual oil was chromatographed on silica gel using 18:1:1 methylene chloride:acetone:methanol as the eluant, to afford 3.29 g (75%) of the product as a waxy solid. NMR ($CDCl_3$) (60 MHz): 1.0-2.2 (m, 9H); 3.4, 3.7 (singlets, total=3H); 3.8-4.2 (m, 1H); 5.0 (br, s, 2H); 5.1-5.7 (m, 2H); 6.2-6.8 (2 multiplets, total=1H); 6.8 (d, J=552Hz, 1H); 7.1 (s, 5H) ppm.

BAD ORIGINAL

## EXAMPLE 21

N-BOC-1-amino-2-(1,2,3,6-tetrahydrophenyl)ethylphosphinic acid methyl ester

A solution of 1.50 g (0.008 moles) of 1-amino-2-(1,2,3,6-tetrahydrophenyl)ethylphosphonous acid in 16 ml of dioxane and 16 ml of 0.5N NaOH was cooled to 0°C in an ice bath, whereupon 2.00 ml (1.90 g; 0.0087 moles) of di-tert-butyl dicarbonate was added all at once. The reaction mixture was stirred vigorously at 0°C for 5 minutes, then removed from the ice bath and stirred at room temperature for 3 hours. The dioxane was removed *in vacuo* and the aqueous solution acidified to pH=1-2 (hydrion paper) with 1N $KHSO_4$. The mixture was extracted with 2 x 75 ml of ethyl acetate. The combined ethyl acetate was dried over anhydrous $Na_2SO_4$, filtered, and the volatiles evaporated *in vacuo* to give a viscous oil.

The residual oil was dissolved in 50 ml of ethyl acetate and treated with 40 ml of ethereal diazomethane solution. The reaction mixture was stirred at room temperature for 1 hour and 10 minutes, then the volatiles were evaporated completely *in vacuo*. The residual oil was chromatographed on silica gel using 25:1:1 methylene chloride:acetone: methanol to afford 1.83 g (95%) of the product as a very thick yellow oil that crystallized very slowly. NMR ($CDCl_3$) (60 MHz): 1.0-2.2 (m containing 9H s at 1.4, total=19H); 3.7, 3.9 (singlets, total=3H); 3.9-4.2 (m, 1H); 5.0-5.7 (m, 2H) ppm.

EXAMPLE 22

Methyl [N-BOC-1-amino-2-(1,2,3,6-tetrahydro)phenylethyl] 2-carbomethoxy-4-methylpentylphosphinate

A solution of 0.700 g (0.003 moles) of the product of Example 21 in 5 ml of absolute methanol was cooled to 0°C whereupon 1.65 ml (0.0033 moles) of 2N NaOMe in methanol was added over a one minute period. The reaction mixture was stirred at 0°C for 10 minutes at which time 0.447 g (0.0032 moles) of methyl 2-(2-methylpropyl)acrylate was added all in one portion. The reaction was stirred at 0°C for 30 minutes, then removed from the ice bath and stirred at room temperature for 6.5 hours. The methanol was removed in vacuo and the residue treated with 30 ml of 1N HCl. The aqueous mixture was extracted with 3 x 25 ml of ethyl acetate. The combined ethyl acetate layers were dried over anhydrous $MgSO_4$, filtered, and the volatiles evaporated in vacuo. The residual oil was purified by chromatography on silica gel using 1% methanol in ethyl acetate as the eluant to afford 0.929 g (82%) of the product as a very thick, colorless oil. NMR ($CDCl_3$) (60 MHz): 0.8-1.0 (m, 6H); 1.0-2.2 (m containing 9H s at 1.5, total=24H); 3.5-3.9 (m, 7H); 4.1-4.5 (v br s, 1H); 5.2-5.7 (m, 2H) ppm.

EXAMPLE 23

Methyl [1-amino-2-(1,2,3,6-tetrahydro)phenylethyl] 2-carbomethoxy-4-methylpentylphosphinate hydrochloride

A solution of 0.324 g (0.00087 moles) of the product of Example 22 in 10 ml of absolute methanol was treated with 2.0 ml of HCl in methanol (144 g/400 ml). The reaction mixture was stirred at room

temperature for 4 hours and monitored by thin layer chromatography. After all of the starting material had disappeared, the volatiles were evaporated completely *in vacuo*, and the residue evacuated to high vacuum to remove residual hydrogen chloride. This material was used directly in the next reaction. $R_f$=0.64 (5:4:1 methylene chloride: acetone:methanol).

## EXAMPLE 24

Methyl [N-(N-BOC-glycylglycyl)-1-amino-2-(1,2,3,6-tetrahydro)phenylethyl] 2-carbomethoxy-4-methylpentylphosphinate

A suspension of 0.202 g (0.00087 moles) of N-BOC-glycylglycine and 0.272 g (0.00096 moles) of disuccinimidyl oxalate in 13 ml of dry acetonitrile was treated with 0.078 ml (0.076 g; 0.00096 moles) of dry pyridine. The heterogeneous mixture was stirred at room temperature under nitrogen atmosphere and slowly became homogeneous over a period of several hours. The reaction was stirred at room temperature for a total for 17 hours. At this time, a solution of the product of Example 23 in 14 ml of acetonitrile containing 0.484 ml (0.352 g; 0.0035 moles) of triethylamine was added all in one portion. The reaction mixture was allowed to stir at room temperature under nitrogen atmosphere for 48 hours. At this time the volatiles were evaporated *in vacuo* and the residue dissolved in 30 ml of ethyl acetate. The organic solution was washed with 2 x 20 ml of saturated $NaHCO_3$, dried over anhydrous $Na_2SO_4$, filtered, and the volatiles removed *in vacuo*. The residue was purified by silica gel chromatography

using 18:1:1 methylene chloride:acetone:methanol as the eluant to afford the product as a light yellow oil. NMR ($CDCl_3$) (300 MHz): 0.8-1.0 (m, 6H); 1.1-1.9 (m containing 9H s at 1.5, total=22H); 1.9 (br s, 1H); 2.2 (m, 1H); 2.9 (m, 1H); 3.6-3.8 (series of singlets; total=6H); 3.7-4.2 (m, 4H); 4.5 (m, 1H); 5.3-5.8 (m, 2H); 6.7-7.2 (m, 2H) ppm.

EXAMPLE 25

Diethyl 2-(N-Benzyl)amino-3-phenylethylphosphonate

A solution of 45.80 g (0.427 moles) of benzylamine in 50 ml of methylene chloride was agitated during the addition of 51.35 g (0.427 moles) of freshly distilled phenylacetaldehyde. After the addition was half-complete, the reaction mixture became cloudy, and after completion of addition the mixture turned a yellow color. At this time, 40 g of anhydrous $Na_2SO_4$ was added and the reaction mixture was stirred at room temperature for 1 hour. The drying agent was filtered off and the filtrate evaporated *in vacuo* to a yellow liquid.

At this time, 56.02 g (0.406 moles) of diethylphosphite was added to the yellow liquid and the mixture was heated in an oil bath maintained at 170°C. The solution was maintained at this temperature for 2.5 hours. The reaction mixture was cooled to room temperature and purified in two approximately equal portions by chromatography on 600 g of silica gel using 2:1 hexanes:ethyl acetate as the eluant. A more mobile impurity came off first, followed by the desired product as a thick yellow liquid. This procedure afforded 59.15 g (42%) of the diester. NMR

($CDCl_3$) (60 MHz): 1.0-1.6 (overlapping t, total=6H); 1.8 (br s, 2H); 2.0 (s, 2H); 2.5-3.5 (m, 2H); 3.7-4.5 (two overlapping q, 4H); 7.0 (m, 10H) ppm.

## EXAMPLE 26

Diethyl 2-(N-Benzyl)amino-3-phenylethylphosphonate hydrochloride

A solution of 30.00 g (0.086 moles) of the product of Example 25 in 650 ml of anhydrous ether was treated with HCl gas for 45 minutes. The reaction was allowed to stand overnight and then filtered to remove a small amount of solid material. The remaining 29.15 g (0.084 moles) of starting material was dissolved in 430 ml of anhydrous ether and treated similarly. The combined filtrates from the two reactions was evaporated *in vacuo* and the viscous oil treated with several 200 ml portions of carbon tetrachloride followed by evaporation *in vacuo*. This procedure led to a sticky crystalline mass which was triturated with anhydrous ethyl ether and filtered and then vacuum dried to afford 41.55 g (64%) of the salt as a white crystalline solid. NMR ($CDCl_3$) (60 MHz): 1.0-1.6 (m, 6H); 2.1 (s, 2H); 3.2-4.5 (m, 9H); 7.2 (s, 10H) ppm.

## EXAMPLE 27

Diethyl 2-Amino-3-phenylethylphosphonate hydrochloride

A solution of 2.00 g (0.0052 moles) of the product of Example 26 in 8 ml of absolute ethanol containing 0.200 g of 10% Pd on carbon was hydrogenated in a Parr type apparatus for 7 hours at 40 psig of hydrogen. The reaction mixture was filtered

through a celite pad and the pad washed well with ethanol. The combined filtrates were evaporated in vacuo to give 1.50 g (99%) of the product as a very viscous, colorless oil. NMR ($d_2$-acetone) (60 MHz): 1.0-1.6 (overlapping t, 6H); 3.3-5.0 (m, 7H); 7.0-7.6 (m, 5H) ppm.

EXAMPLE 28

Diethyl 2-Amino-3-cyclohexylethylphosphonate hydrochloride

A solution of 1.50 g (0.0051 moles) of the product of Example 26 from a procedure similar to Example 27 in 20 ml of glacial acetic acid was treated with 1.50 g of $PtO_2$ and hydrogenated at 60°C and 50 psig of hydrogen in a Parr type of apparatus. After 2 hours, the reaction mixture was cooled to room temperature, filtered through a celite pad and the pad washed well with glacial acetic acid. The filtrate was evaporated completely in vacuo to give 1.34 g (86%) of essentially pure product. NMR ($CDCl_3$) (60 MHz): 0.8-2.2 (m, 19H); 3.6 (br s, 1H); 3.9-4.5 (m, 4H); 8.6 (v br s, 3H) ppm.

EXAMPLE 29

Methyl 2-(2-methylpropyl)acrylate

This compound was prepared by the method of J. Harley-Mason et al. (Tetrahedron 1980, 36, 1063) in approximately 35% overall yield. NMR ($CDCl_3$) (300 MHz): 0.9 (d, 6H); 1.8 (septet, 1H); 2.2 (d, 2H); 3.7 (s, 3H); 5.5 (d, 1H); 6.1 (d, 1H) ppm.

## EXAMPLE 30

Methyl (N-CBZ-1-amino-2-cyclohexylethyl) carbomethoxymethylphosphinate

To a mixture of benzylcarbamate (8.66 g, 0.057 mol), pivalic acid (11.72 g, 0.11 mol) and 11 g of predried powdered molecular sieves in 120 ml of dry toluene was added carbomethoxymethyldichlorophosphine (10 g, 0.057 mol). The reaction mixture was cooled to 0°C and to it was added dropwise cyclohexylacetaldehyde (7.2 g, 0.057 mol). After stirring for 30 minutes at 0°C and 2 hours at room temperature, the reaction was filtered and the solvents removed by evaporation *in vacuo*. The residue was redissolved in 100 ml of dichloromethane cooled to 0°C and esterified with an ether solution of diazomethane. The solvents and excess diazomethane were subsequently removed by evaporation *in vacuo* and the crude product was purified by chromatography to give 11 g of the title compound.

Chromatography: silica, ethyl acetate

TLC (silica, ethyl acetate) $R_f$=0.48

NMR ($CDCl_3$, TMS) 0.9-2.0 (m, 13H), 2.95 (d 16Hz, 2H), 3.64 (s, 3H), 3.7 (d 12Hz, 3H), 3.9-4.5 (m.1H), 5.1 (s,2H), 5.4 and 5.8 (d 10Hz, 1H), 7.2 (s.5H)

mass spectrum: $M^+$

## EXAMPLE 31

Methyl (N-CBZ-1-amino-3-methylbutyl) carbomethoxymethylphosphinate

This ester is prepared by the procedure described in Example 33, using 3-methylbutyraldehyde in place of cyclohexylacetaldehyde. It can also be prepared by the method of P. A. Bartlett *et al.*, *J. Amer. Chem. Soc.*, 106, 4282-83 (1984).

EXAMPLE 32

N-CBZ-1-aminoethylphosphinic acid

The title compound was prepared using the procedure described in Example 2 with paraldehyde replacing hexahydrophenylacetaldehyde.

EXAMPLE 33

Methyl (N-BOC-1-amino-2-cyclohexylethyl) 2-carbomethoxy-4-methyl-(E & Z)-2-butenyl phosphinate

A solution of 0.504 g (1.64 mmol) the phosphinic ester product from Example 4 in dry methanol (2.5 ml) at 0°C was treated dropwise over ten minutes with 0.90 ml of 2.0N methanolic sodium methoxide (1.8 mmol, 1.1 eq.). When addition of the base was complete, 0.38 ml (0.48 g, 2.44 mmol, 1.5 eq.) of trimethyl 2-phosphonoacrylate was added dropwise over 2 minutes. The mixture was warmed to room temperature and stirred for 30 minutes. At this time, tlc analysis (ethyl acetate/acetonitrile/methanol 9:1:.5; E. Merck .25 mm silica plates) indicated complete disappearance of starting phosphinic ester ($R_f$ .8) with formation of a new more polar material with $R_f$ of .3.

The mixture was re-cooled to 0°C and 0.30 ml (0.24 g, 3.3 mmol, 2.0 eq.) of distilled isobutyraldehyde was added dropwise over 2 minutes. The mixture was warmed to room temperature when the addition of the aldehyde was complete. After 1 hour, tlc analysis (as above) indicated complete disappearance of the polar intermediate and formation of a new UV-active material.

The mixture was diluted with ethyl acetate to a total volume of 30 ml and washed with pH 7.0 phosphate buffer (2 x 10 ml). The organic layer was separated and washed once with saturated aqueous sodium chloride (5 ml) and dried ($MgSO_4$). After filtration and removal of volatiles in vacuo, the crude product was purified by medium pressure liquid chromatography (10:1 EtOAc:$CH_3CN$). Two major products were isolated; fraction A (0.190 g, 0.43 mmol, 26%) and fraction B (0.217 g, 0.49 mmol, 30%). Product A, which eluted from the column first, was identified as the E isomer by analysis of its 300 MHz proton NMR spectrum (olefinic proton resonance at 6.68 ppm ($CDCl_3$). Product B is the Z isomer (olefinic proton resonance at 6.03 ppm).

Anal. Calcd for $C_{22}H_{40}NO_6P$:

| | | |
|---|---|---|
| | | C, 59.31; H, 9.05; N, 3.14. |
| Found: | (Fraction A) | C, 59.07; H, 8.86; N, 3.08; |
| | (Fraction B) | C, 59.47; H, 8.82; N, 3.15. |

MS (FAB) 446 (both isomers) ($M^+$ +1).

## EXAMPLE 34

Methyl 2-(2-methylpropyl)acrylate

This compound was prepared by the method of J. Harley-Mason et al. (Tetrahedron 1980, 36, 1063) in approximately 35% overall yield. NMR ($CDCl_3$) (300 MHz): 0.9 (d, 6H); 1.8 (septet, 1H); 2.2 (d, 2H); 3.7 (s, 3H); 5.5 (d, 1H); 6.1 (d, 1H) ppm.

EXAMPLE 35

Methyl 2-(cyclohexylmethyl)acrylate

This compound was prepared as above in approximately 20% overall yield from dimethyl malonate and bromomethylcyclohexane. NMR ($CDCl_3$) (60 MHz): 0.8-2.0 (m, 11H); 2.2 (d, 2H); 3.7 (s, 3H); 5.4 (m, 1H); 6.0 (d, J=2Hz, 1H) ppm.

EXAMPLE 36

Methyl 2-(n-propyl)acrylate

This compound was prepared as above in approximately 45% overall yield from dimethyl malonate and n-propyl bromide. bp.=51-53°C at 20 mm of Hg. NMR ($CDCl_3$) (60 MHz): 0-.9 (t, J=7Hz, 3H); 1.2-1.8 (m, 2H); 2.3 (t, J=7Hz, 2H); 3.8 (s, 3H); 5.5 (m, 1H); 6.1 (br s, 1H) ppm.

EXAMPLE 37

Methyl 2-(2-propyl)acrylate

This compound was prepared as above in approximately 10% overall yield from dimethyl malonate and isopropyl bromide. bp.=130-132°C at $P_{atm}$; NMR ($CDCl_3$) (60 MHz): 1.1 (d, J=7Hz, 6H); 2.8 (septet, 1H); 3.8 (s, 1H); 5.4 (t, 1H); 6.0 (br s, 1H) ppm.

EXAMPLE 38

Methyl [N-(N-(N-BOC-2-amino-3-(1-naphthyl)propionyl)-histidyl)1-amino-2-cyclo-·exylethyl]2-carbomethoxy-4-methylpentylphosphinate

A solution of 0.300 g (0.32 mmol) of the product of Example 14 in 5 ml of dry dimethylformamide was put in a Fisher-Porter tube and treated with ca. 20 ml of anhydrous ammonia. The tube was sealed and the reaction was allowed to stir at room temperature for 8.25 hours. The tube was opened and the ammonia allowed to evaporate. The residue was transferred to a flask and the volatiles removed completely in vacuo. The dark residue was triturated several times with 4 ml portions of anhydrous ether. This afforded the desired product as a yellow powder (0.133 g; 54%). The mother liquors were evaporated and the residue chromatographed using mplc and 18:1:1 methylene chloride:acetone:methanol as the eluant to afford an additional 24 mg (10%) of the product as a crystalline solid. 28H); 2.7-3.2 (m, 6H); 3.5-3.8 (m, 6H); 4.1-5.4 (m, 3H); 6.8-7.6 (m, 6H); 7.6-7.9 (m, 2H); 8.0 (br s, 1H) ppm.

## EXAMPLE 39

Methyl (N-CBZ-1-amino-2-cyclohexyl)2-carbomethoxy-3-cyclohexylpropylphosphinate

A solution of 3.39 g (0.010 moles) of the product of Example 3 in 25 ml of absolute methanol was cooled to 0C under $N_2$. At this time, a solution of 5.50 ml of 2N NaOMe in methanol was added dropwise over a period of ca. 1 min. The reaction mixture was stirred for 10 min. and at this time, 1.91 g (0.0105 moles) of the product of Example 35 was added over a 1 min. period. The reaction mixture was stirred at 0°C for 30 min., then at room temperature for 23 hours. The volatiles were removed in vacuo and the residue added to 50 ml of ice-cold 1N HCl. The mixture was extracted with 3 X 50 ml of

ethyl acetate. The combined ethyl acetate was dried over anh. $MgSO_4$, filtered, and the volatiles evaporated *in vacuo* to a colorless oil. The residue was chromatographed on silica gel using 18:1:1 methylene chloride:acetone:methanol as the eluant to afford 4.26 g (82%) of the desired product as a very viscous oil. NMR ($CDCl_3$) (300 MHz): 0.7-1.9 (m, 28H); 2.1 (m, 1H); 2.9 (br s, 1H); 3.6-3.8 (m, 6H); 4.1 (br s, 1H); 5.1 (s, 2H); 7.3 (s, 5H) ppm.

## EXAMPLE 40

Methyl (1-amino-2-cyclohexyl)2-carbomethoxy-3-cyclohexylpropylphosphinate

A mixture of 4.00 g (7.7 mmol) of the product of Example 39 in 50 ml of absolute methanol was treated with 1.00 g of 10% Pd on C and hydrogenated in a Parr apparatus at 50 psig of $H_2$ for 20 hours. The catalyst was filtered off through celite and the pad washed well with methanol. The filtrate was evaporated *in vacuo* and the residue was chromatographed on silica gel using 18:1:1 methylene chloride:acetone:methanol as the eluant. This afforded 2.20 g (74%) of the desired product as a viscous oil. NMR ($CDCl_3$) (300 MHz): 0.7-1.9 (m, 28H); 2.1 (t, 1H); 2.2 (m, 1H); 2.9 (br s, 2H); 3.7 (m, 6H) ppm.

EXAMPLE 41

Methyl [N-(N-BOC-$N^{im}$-DNP-histidyl)-1-amino-2-cyclohexylethyl] 2-carbomethoxy-3-cyclohexylpropylphosphinate

A mixture of 2.00 g (5.2 mmol) of the product of Example 40, 2.29 g (5.2 mmol) of N-BOC-$N^{im}$-(DNP)-histidine, and 0.768 g (5.7 mmol) of HOBT in 50 ml of dry methylene chloride was stirred until it was homogeneous. At this time, the reaction mixture was cooled to 0°C in an ice bath and 1.18 g (5.7 mmol) of DCC was added all at once. The reaction mixture was stirred under $N_2$ and allowed to warm to room temperature of its own accord. The mixture slowly became heterogeneous as a white powder precipitated out. After 20 hours the reaction mixture was diluted with 100 ml of anhydrous ether and filtered through celite. The filtrate was evaporated *in vacuo* and the residue was dissolved in 100 ml of methylene chloride. The organic solution was washed with 2 X 50 ml of saturated $NaHCO_3$, dried over anhydrous $Na_2SO_4$, filtered and the volatiles evaporated *in vacuo*. The residue was chromatographed on silica gel using 18:1:1 methylene chloride:acetone:methanol as the eluant to afford 2.69 g (65%) of the desired product as a viscous dark yellow oil. NMR ($CDCl_3$) (300 MHz): 0.7-1.9 (m containing 9H s at 1.4, 37H); 2.8 (br s, 1H); 3.1 (m, 1H); 3.7 (m, 6H); 4.4 (br s, 1H); 6.0 (m, 1H); 7.0 (m, 1H); 7.5-8.0 (m, 2H); 8.6 (dd, 1H); 8.9 (s, 1H) ppm.

EXAMPLE 42

Methyl [N-($N^{im}$-DNP-histidyl)-1-amino-2-cyclohexylethyl] 2-carbomethoxy-3-cyclo-hexylpropylphosphinate

A solution of 2.00 g (2.6 mmol) of the product of Example 41 in 20 ml of methanol was treated all at once with 20 ml of HCl in methanol (134.4 g of HCl in 400 ml of methanol). The reaction mixture was stirred at room temperature for 1 hour 15 minutes. The volatiles were removed completely *in vacuo*, and the residue triturated with 2 X 15 ml of anhydrous ether to afford 1.83 g (99%) of the desired product. NMR ($CDCl_3$) (300 MHz): 0.7-2.0 (m, 28H); 2.2 (m, 1H); 2.8 (m, 1H); 3.4-3.9 (m, 6H); 4.3 (br s, 1H); 4.8 (br s, 1H); 7.4-9.6 (m, 8H) ppm.

EXAMPLE 43

Methyl [N-(N-(N-CBZ-2-amino-3-(1-naphthyl)propionyl)-$N^{im}$-DNP-histidyl)-1-amino-2-cyclohexylethyl]2-carbomethoxy-3-cyclohexylpropylphosphinate

A mixture of 0.353 g (1.00 mmol) of the product of Example 11 and 0.312 g (1.1 mmol) of disuccinimidyl oxalate in 10 ml of dry acetonitrile was treated with 0.087 g (0.09 ml; 1.1 mmol) of pyridine. The reaction mixture was stirred at room temperature under $N_2$ for 16 hours. The mixture turned from heterogeneous to homogeneous during this period. At this time, a mixture of 0.727 g (0.001 moles) of the product of Example 42 and 0.404 g (4.0 mmol; 0.56 ml) of triethylamine in 10 ml of acetonitrile was added all at once. The mixture turned darker and was stirred at room temperature under $N_2$ for 24 hours. At this time, the volatiles were

evaporated completely in vacuo and the residue dissolved in 50 ml of methylene chloride. The organic solution was washed with 2 X 20 ml of saturated $NaHCO_3$, dried over anhydrous $Na_2SO_4$, filtered, and the volatiles evaporated in vacuo. The residue was chromatographed on silica gel using 18:1:1 methylene chloride:acetone:methanol as the eluant to afford 0.631 g (62%) of the desired product as a glassy foam. NMR ($CDCl_3$) (300 MHz): 0.4-1.9 (m, 28H); 2.0-2.2 (m, 1H); 2.3-3.2 (m, 2H); 3.6-3.8 (m, 6H); 4.2-4.8 (m, 2H); 5.1 (d, 2H); 5.3-5.8 (m, 2H); 6.7-8.3 (m, 15H); 8.5 (dt, 2H); 8.8 (q, 1H) ppm.

EXAMPLE 44

Methyl [N-(N-(N-BOC-2-amino-3-(1-naphthyl)propionyl)-$N^{im}$-DNP-histidyl)-1-amino-2-cyclohexylethyl] 2-carbomethoxy-3-cyclohexylpropylphosphinate

A mixture of 0.315 g (1.00 mmol) of the product of Example 12 and 0.312 g (0.0011 moles) of disuccinimidyl oxalate in 10 ml of dry acetonitrile was treated with 0.087 g (1.1 mmol; 0.09 ml) of pyridine. The reaction mixture was allowed to stir at room temperature under $N_2$ for 16 hours. During this period the reaction went from heterogeneous to homogeneous. At this time, a mixture of 0.727 g (1.00 mmol) of the product of Example 42 and 0.404 g (4.00 mmol; 0.56 ml0 of triethylamine in 10 ml of acetonitrile was added all at once. The reaction mixture turned darker and stirring at room temperature under $N_2$ was continued for 24 hours. At this time, the volatiles were removed in vacuo and the residue dissolved in 50 ml of methylene

chloride. The organic solution was washed with 2 X 50 ml of saturated $NaHCO_3$, dried over anhydrous $Na_2SO_4$, filtered, and the volatiles removed <u>in vacuo</u>. The residue was chromatographed on silica gel using 18:1:1 methylene chloride:acetone:methanol as the eluant to give 0.571 g (58%) of the desired product as a glassy foam. NMR ($CDCl_3$) (300 MHz)-: 0.7-1.9 (m, containing 9H s at 1.2, 37H); 2.7-3.3 (m, 4H); 3.5-3.8 (m, 6H); 4.4 (m, 3H); 4.7 (m, 1H); 5.0 (m, 1H); 6.8-8.0 (m, 9H); 8.2 (d, 2H); 8.6 (dd, 2H); 8.8 (t, 1H) ppm.

## EXAMPLE 45

Methyl [N-(N-(N-CBZ-2-amino-4-phenylbutyryl)-$N^{im}$-DNP-histidyl)-1-amino-2-cyclo-hexylethyl] 2-carbomethoxy-4-methylpentylphosphinate

A mixture of 0.313 g (1.00 mmol) of N-CBZ-2-amino-4-phenylbutyric acid and 0.312 g (1.1 mmol) of disuccinimidyl oxalate in 20 ml of dry acetonitrile was treated with 0.087 g (1.1 mmol; 0.90 ml) of pyridine. The reaction mixture was stirred at room temperature under $N_2$ for 24 hours. During this period the reaction went from heterogeneous to homogeneous. At this time a mixture of 0.687 g (0.001 moles) of the product of Example 10 and 404 mg (4.00 mmol; 0.56 ml) of triethylamine in 10 ml of acetonitrile was added all at once. The reaction mixture was stirred at room temperature under $N_2$ for 30 hours. The volatiles were evaporated completely <u>in vacuo</u> and the residue dissolved in 50 ml of methylene chloride. The organic solution was washed with 2 X 15 ml of saturated $NaHCO_3$, dried

over anhydrous $Na_2SO_4$, filtered and evaporated _in vacuo_. The residue was chromatographed on silica gel using 18:1:1 methylene chloride:acetone:methanol to give 0.363 g (38%) of the desired product as a glassy foam. NMR ($CDCl_3$) (300 MHz): 0.7-0.9 (m, 6H); 0.9-2.2 (m, 21H); 2.6 (m, 2H); 2.7-3.2 (m, 3H); 3.4-3.7 (m, 6H); 4.1 (m, 1H); 4.3 (m, 1H); 4.7 (m, 1H); 5.0 (q, 2H); 5.3 (m, 1H); 6.8-8.2 (m; 13H); 8.4 (m, 2H); 8.8 (m, 1H) ppm.

## EXAMPLE 46

Methyl [N-(N-(N-BOC-2-amino-4-phenylbutyryl)-$N^{im}$-DNP-histidyl)-1-amino-2-cyclo-hexylethyl] 2-carbomethoxy-4-methylpentylphosphinate

A mixture of 0.279 g (1.00 mmol) of N-BOC-2-amino-4-phenylbutyric acid and 0.312 g (0.0011 moles) of disuccinimidyl oxalate in 20 ml of dry acetonitrile was treated with 0.087 g (1.1 mmol; 0.90 ml) of pyridine. The reaction mixture was stirred at room temperature under $N_2$ for 24 hours. During this period the reaction mixture went from heterogeneous to homogeneous. At this time, a mixture of 0.687 g (1.00 mmol) of the product of Example 10 and 404 mg (4.00 mmol; 0.56 ml) of triethylamine in 10 ml of acetonitrile was added all at once. The reaction mixture turned darker and was stirred at room temperature under $N_2$ for 30 hours. The volatiles were removed _in vacuo_ and the residue was dissolved in 50 ml of methylene chloride. The organic mixture was washed with 2 X 15 ml of saturated $NaHCO_3$, dried over anhydrous $Na_2SO_4$, filtered, and the volatiles evaporated _in vacuo_. The

residue was chromatographed on silica gel using 18:1:1 methylene chloride:acetone:methanol as the eluant. This provided 0.493 g (54%) of the desired product as a glassy foam. NMR ($CDCl_3$) (300 MHz): 0.7-1.0 (m, 6H); 1.0-2.2 (m containing 9H s at 1.5, 28H), 2.6 (m, 2H); 2.7-3.3 (m, 3H); 3.5-3.8 (m, 8H); 4.0 (m, 2H); 4.4 (m, 1H); 4.7 (m, 1H); 5.0 (m, 1H); 6.9-8.0 (m, 7H); 8.5 (m, 2H); 8.8 (m, 1H) ppm.

## EXAMPLE 47

Methyl [N-(N-(N-CBZ-phenylalanyl)-$N^{im}$-DNP-histidyl)-1-amino-2-cyclohexylethyl]-2-carbomethoxy-4-methylpentylphosphinate

A mixture of 0.156 g (0.55 mmol) of disuccinimidyl oxalate and 0.150 g (0.0005 moles) of N-CBZ-phenylalanine in 10 ml of dry acetonitrile was treated with 0.04 ml (0.5 mmol) of pyridine. The reaction mixture was stirred at room temperature under $N_2$ for 2 hours. At this time, an additional 0.010 g of disuccinimidyl oxalate was added and stirring under $N_2$ continued for 5 hours. During this time the mixture went from heterogeneous to homogeneous. At this time, 0.330 g the product of Example 10 was added, immediately followed by 0.202 g (2.00 mmol; 0.28 ml) of triethylamine. The reaction mixture immediately turned darker. The mixture was stirred at room temperature for 18h. At this time the volatiles were evaporated completely *in vacuo* and the residue dissolved in 25 ml of ethyl acetate. The organic solution was washed with 3 X 15 ml of saturated $NaHCO_3$, dried over anhydrous $Na_2SO_4$, filtered and the ethyl acetate evaporated *in vacuo*.

The residue was chromatographed on silica gel using 18:1:1 methylene chloride:acetone:methanol as the eluant to afford 0.250 g (55%) of the desired product as a glassy foam. NMR ($CDCl_3$) (300 MHz): 0.7-1.0 (m, 6H); 1.0-2.0 (m, 19H); 2.5-3.4 (m, 5H); 3.5-3.7 (m, 6H); 4.0 (br s, 1H); 4.3 (br s, 1H); 5.1 (d, 2H); 5.3 ( m, 1H); 7.1 (m, 2H); 7.2-7.4 (m, 10H); 7.5-8.4 (m, 2H); 8.5 (m, 2H); 8.8 (m, 1H) ppm.

EXAMPLE 48

Methyl [N-(N-(N-BOC-phenylalanyl)-$N^{im}$-DNP-histidyl)-1-amino-2-cyclohexylethyl]-2-carbomethoxy-4-methylpentylphosphinate

A mixture of 0.156 g (0.55 mmol) of disuccinimidyl oxalate and 0.133 g (0.50 mmol) of N-BOC-phenylalanine in 10 ml of dry acetonitrile was treated with 0.04 ml (0.50 mmol) of pyridine. The reaction mixture was stirred at room temperature under $N_2$ for 3 hours. At this time, an additional 0.010 g of disuccinimidyl oxalate was added and stirring under $N_2$ continued for 5 hours. During this time the mixture went from heterogeneous to homogeneous. At this time, 0.330 g (0.50 mmol) of the product of Example 10 was added, immediately followed by 0.202 g (2.00 mmol; 0.28 ml) of triethylamine. The reaction mixture immediately turned darker. The mixture was stirred at room temperature for 18.5 hours. At this time the volatiles were evaporated completely *in vacuo* and the residue dissolved in 20 ml of ethyl acetate. The organic solution was washed with 3 X 15 ml of saturated $NaHCO_3$, dried over anhydrous $Na_2SO_4$,

filtered and the ethyl acetate evaporated *in vacuo*. The residue was chromatographed on silica gel using 18:1:1 methylene chloride:acetone:methanol as the eluant to afford 0.266 g (61%) of the desired product as a glassy foam. NMR ($CDCl_3$) (300 MHz): 0.7-0.9 (m, 6H); 0.9-2.0 (m containing 3H s at 1.3, 26H); 2.5-3.5 (m, 5H); 3.5-3.7 (m, 6H); 4.0 (br s, 1H); 4.3 (br s, 2H); 4.7 (br s, 1H); 5.0 (m, 1H); 6.8-7.0 (m, 2H); 7.0-7.3 (m, 5H); 7.5-8.0 (m, 2H); 8.6 (m, 2H); 8.9 (m, 1H) ppm.

## EXAMPLE 49

[N-(N-2-Amino-3-(1-naphthyl)propionyl-histidyl)-1-amino-2-cyclohexylethyl] 2-carboxy-4-methylpentyl-phosphinic acid

A solution of 0.045 g (0.055 mmol) of the product of Example 13 in 3 ml of ethanol was treated with 1.10 ml of 0.100N NaOH (aq.). The reaction mixture was stirred at room temperature for 20 hours, at which time an additional 1.00 ml of 0.100N NaOH was added. Stirring was continued for 1 hour, then the volatiles were evaporated completely *in vacuo*. The residue was triturated with 3 X 2 ml of anhydrous ether, and the residue was purified by passing it through a column containing ca. 15 g of DOWEX 50W-X4 hydrogen form ion-exchange resin (5.2 meq/dry gram). This afforded 0.007 g (15%) of the desired product as a very light yellow glassy foam. NMR ($CD_3OD$) (300 MHz): 0.6-0.9 (m, 6H); 1.1-2.0 (m, 20H); 2.7 (m, 2H); 3.6 (m, 3H); 4.2 (br s, 1H); 4.4 (m, 1H); 4.6 (m, 1H); 7.2-7.5 (4H); 7.7 (d, 1H); 7.8 (d, 1H); 8.1 (m, 1H) ppm.

EXAMPLE 50

Methyl [N-(N-(N-CBZ-2-amino-3-(1-naphthyl)propionyl)-histidyl)-1-amino-2-cyclo-hexylethyl] 2-carbomethoxy-3-cyclohexylpropylphosphinate

A solution of 0.350 g (0.34 mmol) of the product of Example 43 in 4 ml of dry dimethylformamide was treated with ca. 15 ml of anhydrous ammonia in a Fisher-Porter Tube. The tube was sealed and the reaction mixture stirred at room temperature for 21 hours. During this time the reaction color went from dark purple to reddish brown. The tube was opened and the ammonia allowed to evaporate of its own accord. The remaining volatiles were evaporated completely *in vacuo* and the residue triturated with anhydrous ether until a free flowing crystalline solid was obtained.

EXAMPLE 51

Methyl [N-(N-(N-BOC-2-amino-3-(1-naphthyl)propionyl)-histidyl)-1-amino-2-cyclo-hexylethyl] 2-carbomethoxy-3-cyclohexylpropylphosphinate

A solution of 0.350 g (0.36 mmol) of the product of Example 44 in 4 ml of dry dimethylformamide was treated with ca. 15 ml of anhydrous ammonia in a Fisher-Porter Tube. The tube was sealed and the reaction mixture stirred at room temperature for 21 hours. During this time the reaction color went from dark purple to reddish brown. The tube was opened and the ammonia allowed to evaporate of its own accord. The remaining volatiles were evaporated completely *in vacuo* and the residue triturated with anhydrous ether until a free flowing crystalline solid was obtained.

EXAMPLE 52

Methyl [N-(N-(N-CBZ-2-amino-4-phenyl)butyryl-histidyl)-1-amino-2-cyclohexyl-ethyl] 2-carbomethoxy-4-methylpentylphosphinate

A solution of 0.200 g (0.21 mmol) of the product of Example 45 in 2 ml of dry dimethylformamide was treated with ca. 12 ml of anhydrous ammonia in a Fisher-Porter Tube. The tube was sealed and the reaction mixture stirred at room temperature for 21 hours. During this period the color of the reaction went from dark purple to reddish-brown. At this time the tube was opened and the ammonia allowed to evaporate of its own accord. The remaining volatiles were evaporated completely in vacuo and the residue was triturated with anhydrous ether until a free flowing solid was obtained.

EXAMPLE 53

Methyl [N-(N-(N-BOC-2-amino-4-phenyl)butyryl-histidyl)-1-amino-2-cyclohexyl-ethyl] 2-carbomethoxy-4-methylpentylphosphinate

A solution of 0.200 g (0.22 mmol) of the product of Example 46 in 2 ml of dry methylformamide was treated with ca. 12 ml of anhydrous ammonia in a Fisher-Porter Tube. The tube was sealed and the reaction mixture stirred at room temperature for 21 hours. During this period the color of the reaction went from dark purple to reddish-brown. At this time the tube was opened and the ammonia allowed to evaporate of its own accord. The remaining volatiles were evaporated completely in vacuo and the residue was triturated with anhydrous ether until a free flowing solid was obtained.

EXAMPLE 54

Methyl (N-histidyl-1-amino-2-cyclohexylethyl) 2-carbomethoxy-4-methylpentylphosphinate

A solution of 0.200 g (0.28 mmol) of the product of Example 10 in 2 ml of dimethylformamide was put into a Fisher-Porter tube and ca. 12 ml of ammonia was condensed in. The tube was sealed and the reaction was stirred at room temperature for 16 hours. The tube was opened and the ammonia was allowed to evaporate of its own accord. The remaining volatiles were evaporated completely *in vacuo* and the residue triturated with anhydrous ether. This afforded the desired product as a yellow solid after purification by chromatography on DOWEX 50W-X4.

EXAMPLE 55

Methyl (N-histidyl-1-amino-2-cyclohexylethyl) 2-carbomethoxy-3-cyclohexylpropylphosphinate

A solution of 0.200 g (0.28 mmol) of the product of Example 42 in 2 ml of dimethylformamide was put into a Fisher-Porter tube and ca. 12 ml of ammonia was condensed in. The tube was sealed and the reaction was stirred at room temperature for 16 hours. The tube was opened and the ammonia was allowed to evaporate of its own accord. The remaining volatiles were evaporated completely *in vacuo* and the residue triturated with anhydrous ether. This afforded the desired product as a yellow solid after purification by chromatography on DOWEX 50W-X4.

EXAMPLE 56

N-Methyl 1-amino-2-cyclohexylphosphinic acid

This acid is prepared using the procedure described in Example 2 with methylamine hydrochloride replacing aminophenylmethane hydrochloride.

EXAMPLE 57

Methyl N-CBZ-N-methyl 1-amino-2-cyclohexylethyl-phosphinate

This ester is prepared from N-methyl-1-amino-2-cyclohexylphosphinic acid using the procedure described in Example 3.

EXAMPLE 58

Diethyl N-methyl-1-amino-2-phenylethylphosphonate

This ester is prepared using the procedure described in Example 25 with equimolar amounts of methyl amine hydrochloride and triethylamine substituted for benzylamine.

EXAMPLE 59

Diethyl N-CBZ-N-methyl-1-amino-2-cyclohexylethyl-phosphonate

This ester is prepared by treatment of the product of Example 58 with benzyl chloroformate and $Et_3N$ in $CH_2Cl_2$ and is purified by silica gel chromatography.

EXAMPLE 60

Diethyl N-CBZ-1-amino-2-cyclohexylethylphosphonate

This ester is prepared by treatment of the product of Example 27 with benzyl chloroformate and $Et_3N$ in $CH_2Cl_2$ and is purified by silica gel chromatography.

EXAMPLE 61

Trimethylacetoxymethyl (N-CBZ-1-amino-2-cyclohexylethyl) 2-carbomethoxy-4-methylpentylphosphinate

Treatment of the product of Example 5 with trimethylsilyl bromide in anhydrous $CH_2Cl_2$, followed by dilution of the mixture into brine and extraction with ethyl acetate, affords (N-CBZ-1-amino-2-cyclohexylethyl)-2-carbomethoxy-4-methylpentylphosphinic acid as a light-yellow foam. A solution of this acid (100 mg) in DMF (0.5 ml) was stirred under nitrogen as $isp_2NEt$ (82 µl) and then a solution of chloromethyl trimethylacetate (71 mg) in DMF (0.5 ml) were added. After 3 days, additional $isp_2NEt$ (82 µl) and chloromethyl trimethylacetate (75 mg) were added. After a total of 4 days, the mixture was diluted with ethyl acetate and washed with 1M citric acid and brine and dried ($MgSO_4$), giving a colorless oil. The product (62 mg, 50%) was isolated by preparative tlc (silica gel) in 1:1 ethyl acetate:hexanes. Mass spectrum (FAB) m/e 582 ($M^+$ +1). 300 MHz NMR ($CDCl_3$) 0.8-2.3 (33H, m); 2.9 (1H, m); 3.7 (3H, s); 4.2 (1H, m); 4.8-5.2 (1H, m); 5.15 (2H, m); 7.35 (5H, s).

EXAMPLE 62

(1-Amino-2-cyclohexylethyl) 2-carbomethoxy-4-methylpentylphosphinic acid

To the product of Example 8 (60 mg; 0.158 mmol) was added HBr-HOAc solution (5 equivalents) and an equivalent volume of HOAc. The solution was stirred for 24 hours and then concentrated to dryness, giving the product (40 mg; 61%). MS: m/e

334 ($M^+$ +1). NMR ($D_2O$): 0.6-2.3 (22H, m); 2.8 (1H, br s); 3.7 (3H, m); 3.3-3.6 (1H, m); 4.2 (1H, m); 8.0 (3H, br s).

EXAMPLE 63

Methyl [N-(N-t-butoxycarbonyl-phenylalanyl)-1-amino-2-cyclohexylethyl] 2-carbomethoxy-4-methylpentylphosphinate

The product of Example 8 (0.26 g) was dissolved in dry $CH_2Cl_2$ (3 ml) and neutralized with $Et_3N$ (0.095 ml) at 0°C. To the above solution were added sequentially N-Boc-phenylalanine (0.22 g), HOBT (0.18 g) and DCC (0.19 g). The mixture after stirring for 4 hours at 0°C was stirred at 25°C for an additional 15 hours. The mixture was filtered, and the $CH_2Cl_2$ phase was washed with $NaHCO_3$ (saturated), water, saturated NaCl and dried ($MgSO_4$). Removal of solvent *in vacuo* gave a foam which was purified by medium pressure liquid chromatography (mplc) using ethyl acetate-hexane (3:1) on a silica gel column yield 0.28 g (70%) (foam). NMR ($CDCl_3$): 7.23 (5H, s), 6.62 (1H, m), 5.15 (1H, m), 4.1-4.41 (2H, m), 3.63 (6H, m), 3.11 (2H, m), 2.85 (2H, m), 0.85-2.05 (32H, m). MS (FAB): m/e 595 ($M^+$ +1).

EXAMPLE 64

[N-(Phenylalanyl)-1-amino-2-cyclohexylethyl] 2-carbomethoxy-4-methylpentylphosphinic acid hydrochloride

The product of Example 63 (39 mg) was treated with trimethylsilylbromide (0.2 ml) in $CH_2Cl_2$ (0.2 ml) at 25°C for 6 hours. Removal of

excess reagent and the solvent in vacuo gave an oil which was dissolved in MeOH (5 ml), stirred for 15 minutes at 25°C and evaporation. The foam, thus obtained, was treated with HCl in dioxane (2N) (0.15 ml) at 25°C for 45 minutes. Removal of excess reagent in vacuo and addition of dry ether gave the product as a white solid (hygroscopic) (25 mg). NMR ($CDCl_3$): 8.95 (1H, bm), 8.32 (1H, m), 7.85 (1H, m), 7.32 (5H, s), 4.05-4.55 (2H, m), 3.62 (3H, m), 3.28 (2H, m), 2.81 (2H, m), 0.80-2.1 (23H, m). MS (FAB): m/e 481 ($M^+$ +1).

## EXAMPLE 65

Methyl [N-(Nα-BOC-Nε-CBZ-lysyl)-1-amino-2-cyclohexylethyl] 2-carbomethoxy-4-methylpentylphosphinate

The free amine obtained from the product of Example 8 (0.39 g) and $Et_3N$ (0.141 ml), was coupled with $N^{\alpha}$-BOC-$N^6$-CBZ-lysine (0.44 g) in presence of HOBT (0.27 g) and DCC (0.25 g) in dry THF at 0°C. The crude product was obtained, as a foam, which was purified by mplc using ethyl acetate-hexane (3:2). Yield 0.45 g (63%). NMR ($CDCl_3$): 7.34 (5H, s), 6.75 (1H, m), 5.36 (1H, m), 5.12 (3H, m), 4.42 (1H, m), 4.10 (1H, m), 3.66 (6H, m), 3.16 (2H, q), 2.88 (2H, m), 0.88-2.15 (38H, m). MS (FAB): m/e 710 ($M^+$ +1); m/e 610 ($M^+$ +1-100).

## EXAMPLE 66

[N-(Lysyl)-1-amino-2-cyclohexylethyl] 2- carboxy-4-methylpentylphosphinic acid

The product of Example 65 (60 mg) was stirred with aqueous 1N NaOH (0.18 ml) in acetone (1 ml) for 24 hours. Removal of acetone in vacuo and

acidification with cold 1N HCl gave the free acid as an oil. The oil was then treated with HBr-AcOH (33%) (1 ml) for 12 hours at 25°C. Excess HBr was removed in vacuo, and the product was precipitated with dry ether. The hygroscopic solid was filtered and dissolved in MeOH (1 ml), and treated with propylene oxide (0.2 ml). The product was finally precipitated with dry ether. The solid was filtered, washed with dry ether and dried to give white powder (39 mg). NMR ($CD_3OD$): 4.18 (2H, m), 2.85 (2H, m), 0.85-2.15 (29H, m). MS (FAB): m/e 498 ($M^+$ +1).

## EXAMPLE 67

Methyl [N-(N-α(N-BOC-2-amino-3-(1-naphthyl)propionyl)-Nε-CBZ-lysyl)-1-amino-2-cyclohexylethyl] 2-carbomethoxy-4-methylpentylphosphinate

The product of Example 65 (0.25 g) was dissolved in 50% TFA in $CH_2Cl_2$ (2 ml), and the mixture was stirred at 25°C for 1-1/2 hours. Removal of excess reagent and $CH_2Cl_2$ in vacuo gave an oil which upon drying over $P_2O_5$ and NaOH in vacuo gave the amine salt as a foam.

The above foam was dissolved in EtOAc (10 ml) and neutralized with saturated $NaHCO_3$. The EtOAc phase was washed with brine and dried over ($Na_2SO_4$). Removal of solvent in vacuo gave the free amine as an oil. The free amine was then coupled with the product of Example 12 (0.18 g) in the presence of HOBT (0.1 g) and DCC (0.11 g) at 0°C in $CH_2Cl_2$ (5 ml). The crude product was purified by flash chromatography using EtOAc-hexane (3:1). Yield 0.25 g (foam). NMR ($CDCl_3$): 6.85-8.1

(13H, m), 6.56 (1H, m), 5.05-5.40 (4H, m), 4.25-4.55 (3H, m), 3.68 (6H, m), 3.12 (2H), 2.85 (2H, m), 0.80-2.20 (39H, m). MS (FAB): m/e 907 ($M^+$ +1).

EXAMPLE 68

Methyl [N-(N-(2-amino-3-(1-naphthyl)propionyl)-N-CBZ-lysyl)-1-amino-2-cyclohexylethyl] 2-carbomethoxy-4-methylpentylphosphinate hydrochloride

The product of Example 67 (60 mg) was dissolved in 50% TFA in $CH_2Cl_2$ (1 ml) at 25°C for 2 hours. Removal of solvent *in vacuo* gave a foam which upon drying *in vacuo* over $P_2O_5$ and NaOH gave a glass-like hygroscopic solid (45 mg). The solid was then treated with 1N HCl in MeOH, and the solvent was removed *in vacuo* to give the desired product as hydrochloride salt (40 mg). NMR ($CDCl_3$): 6.8-8.3 (15H, m), 5.35-5.55 (2H, m), 5.12 (2H, s), 4.45 (2H, m), 4.15 (1H, m), 3.65 (6H, m), 3.10 (2H, m), 2.80 (2H, m), 0.80-2.1 (30H, m). MS (FAB): m/e 807 ($M^+$ +1). FAB-MS: $(M+H)^+$ 807.

EXAMPLE 69

Methyl [N-(N-(N-BOC-2-amino-3-(1-naphthyl)propionyl)-lysyl-1-amino-2-cyclohexylethyl] 2-carbomethoxy-4-methylpentylphosphinate

The product of Example 68 (50 mg) was treated with $H_2$ in the presence of Pd-C (10%) (10 mg) in MeOH (5 ml) (containing AcOH (7 µl)) for 3 hours at 40 psi. The catalyst was filtered off and the filtrate upon evaporation *in vacuo* gave the pure product as the acetate salt (40 mg). NMR ($CDCl_3$): 6.95-8.1 (11H, m), 5.60 (1H, d), 5.22 (1H, m), 4.51

(2H, m), 4.12 (1H, m), 3.68 (6H, m), 2.6-2.95 (4H, m), 2.05 (3H, s), 0.85-2.15 (39H, m). MS (FAB): m/e 773 ($M^+$ +1).

EXAMPLE 70

Methyl (1-amino-2-cyclohexylethyl)carbomethoxymethylphosphinate hydrochloride

The product of Example 30 (3.58 g) was hydrogenated in methanol (30 ml) [containing concentrated HCl (0.72 ml)] over Pd-C (10%) (0.35 g) at 40 psi overnight at 25°C. The catalyst was filtered off and the filtrate was evaporated to give the product as glass-like solid foam (2.67 g) (94%). MS (FAB): m/e 278 ($M^+$ +1).

EXAMPLE 71

Methyl [(N-BOC-phenylalanyl)-1-amino-2-cyclohexylethyl] carbomethoxymethylphosphinate

The product of Example 70 (0.78 g) was treated with $Et_3N$ (0.35 ml) in a mixture of THF-$CH_2Cl_2$ (1:1) (6 ml) at 0°C. The free amine, thus obtained, was coupled with N-BOC-phenylalanine (0.66 g) in presence of DCC (0.56 g) and HOBT (0.5 g) under standard condition. Filtration and processing of the filtrate gave the crude product as a foam which was purified by flash chromatography on silica gel using 10% hexane in ethyl acetate. Yield (1.18 g) (91%) (foam). MS: M+H = 525, M-99 = 425 (-BOC). $^1H$ NMR: 7.35-7.15 (5H, m), 5.15 (1H, br s), 4.90 (1H, br s), 4.6 (1H, m), 4.4 (1H, m), 3.85-3.65 (6H, m), 3.20-3.0 (2H, m), 3.0 (2H, d, J=15Hz), 2.0-1.7 (13H, m), 1.4 (9H, m).

EXAMPLE 72

(N-Phenylalanyl-1-amino-2-cyclohexylethyl) 2-carbomethoxymethylphosphinic acid hydrobromide

The product of Example 71 (43 mg) was treated with HBr-AcOH (33%) (0.15 ml) at 25°C for 16 hours. Excess HBr was removed *in vacuo*, and the residue was dissolved in ethyl acetate (2 ml) and treated with excess propylene oxide. Removal of solvent *in vacuo* and drying over $P_2O_5$ and NaOH gave the titled compound as a glass like solid (24 mg). NMR ($CDCl_3$): 7.9-7.5 (1H, m), 7.35 (5H, m), 4.4-4.0 (3H, m), 3.74 (3H, m), 3.31-2.9 (1H, m), 2.9-2.6 (2H, dd), 2.0-0.8 (13H, m). MS (FAB): m/e 411 ($M^+$ +1).

EXAMPLE 73

Methyl [(Nα-BOC-Nε-CBZ-lysyl)-1-amino-2-cyclohexylethyl] carbomethoxymethylphosphinate

The product of Example 70 (0.75 g) was dissolved in a mixture of dry THF (3 ml) and $CH_2Cl_2$ (3 ml) and neutralized with $Et_3N$ (0.33 ml). $N^{\alpha}$-BOC-N -CBZ-lysine N-hydroxysuccinimide ester (0.39 g) was added to the above solution, and the mixture was stirred at 25°C for 24 hours. The crude product obtained, after concentration of the reaction mixture, was purified by flash chromatography on silica gel using ethyl acetate as the eluent. Yield (0.5 g) (foam). NMR ($CDCl_3$): 7.36 (5H, s), 6.45 (1H, brs), 5.4-4.7 (2H, m), 5.1 (2H, s), 4.6 (1H, m), 4.05 (1H, m), 3.85-3.60 (6H, m), 3.2 (2H, brs), (2H, d, J=10), 1.90-.8 (19H, m), 1.60, 1.40 (9H, s). MS (FAB): m/e 640 ($M^+$ +1).

EXAMPLE 74

Methyl [(Nα-BOC-lysyl)-1-amino-2-cyclohexylethyl] carbomethoxymethylphosphinate

The product of Example 73 (60 mg) was hydrogenated over Pd/C (10%) in MeOH (5 ml) containing 1 equivalent of acetic acid under standard conditions to give the titled compound as a foam (51 mg). NMR ($CDCl_3$): 8.5 (3H, br s), 8.2-7.6 (1H, m), 5.9-5.5 (1H, m), 4.5 (1H, m), 4.1 (1H, br s), 3.7 (6H, m), 3.2-2.8 (4H, m), 2.0 (3H, s), 1.90-.7 (19H, m), 1.4 (9H, s). MS: m/e 506 ($M^+$ +1).

EXAMPLE 75

Methyl [N-(N-(2-naphthyloxy)acetyl-phenylalanyl)-1-amino-2-cyclohexylethyl] carbomethoxymethylphosphinate

The product of Example 71 (0.395 g) was treated with trifluoroacetic acid -$CH_2Cl_2$ (1:1) (5 ml) at 25°C for 2 hours. Removal of excess reagent *in vacuo* gave the trifluoroacetate salt of the free amine as a foam, which was dissolved in dry THF (5 ml) and neutralized with $Et_3N$ (0.12 ml). To the free amine, thus obtained, (2-naphthoxy)-acetic acid N-hydroxysuccinimide ester (0.31 g) was added, and the mixture was stirred at 25°C for 24 hours. The crude product obtained, after removal of solvent *in vacuo*, was purified by mplc using 25% hexane in ethyl acetate. Yield 0.34 g (65%) (foam). MS: M+H = 609. $^1H$ NMR: 7.8-7.0 (12H, m), 6.9 (1H, d, J=15Hz), 4.90 (1H, m), 4.7-4.4 (3H, m), 3.8- .6 (6H, m), 3.25-2.9 (4H, m), 3.0-2.8 (2H, d, J=15Hz), 1.9-0.7 (13H, m).

## EXAMPLE 76

Methyl [N-(N-(N-CBZ-2-amino-3-(2-naphthyl)propionyl-phenylalanyl)-1-amino-2-cyclohexylethyl] carbomethoxymethylphosphinate

The product of Example 71 (0.43 g) was reacted with 50% TFA in $CH_2Cl_2$ (5 ml) at 25°C for 2.5 hours. Removal of excess reagent *in vacuo* gave the corresponding TFA salt as a foam. The above salt was dissolved in dry THF (5 ml) and treated with $Et_3N$ (0.13 ml) followed by the addition of N-CBZ-2-amino-3-(1-naphthyl)propionic acid (0.33 g), HOBT (0.19 g) and DCC (0.21 g). The mixture was stirred at 0°C for 2 hours and at 25°C for 16 hours. The reaction mixture was filtered, and the filtrate was diluted with EtOAc (20 ml) washed with saturated $NaHCO_3$, water and dried ($MgSO_4$). Removal of the solvent gave the crude product as an oil, which was purified by flash chromatography on silica gel using 20% hexane in ethyl acetate. Yield 0.49 g (69%) (foam). $^1$NMR ($CDCl_3$): 8.2-7.0 (17H, m), 6.9-6.2 (2H, m), 5.9-5.5 (1H, m), 5.25-4.90 (4H, m), 4.85-4.6 (1H, m, CH), 4.6-4.2 (3H, m), 3.8-3.3 (6H), 3.0-2.6 (2H, m), 1.9-0.7 (11H, m).

## EXAMPLE 77

Methyl [N-(N-(Nα-CBZ-2-amino-3-(2-naphthyl)propionyl)-Nε-CBZ-lysyl)-1-amino-2-cyclohexylethyl] carbomethoxymethylphosphinate

The product of Example 73 (0.23 g) was treated with 50% TFA-$CH_2Cl_2$ (2.5 ml) at 25°C and the corresponding trifluoroacetate salt of the amine was obtained as described before. The salt was dissolved in dry THF (4 ml), neutralized with $Et_3N$

(0.05 ml) and coupled with the product of Example 11 (0.125 g) in the presence of HOBT (0.072 g) and DCC (0.081 g) under standard conditions. The reaction was filtered, and the filtrate was washed with saturated $NaHCO_3$, water and dried ($MgSO_4$). Removal of the solvent gave an oil which was purified by mplc using 20% hexane in ethyl acetate. Yield 0.21 (68%). $^1$H NMR: 8.3-7.1 (17H, m), 7.0-5.2 (4H, m), 5.1 (4H, m), 4.4-4.0 (2H, m), 4.5 (2H, m), 3.8-3.4 (6H, m), 3.1-2.8 (4H, m), 1.9-.6 (19H, m).

Claims to the invention follow.

WHAT IS CLAIMED IS:

1. A compound of the formula

A-E-G

wherein

A is hydrogen, or $R_a$-, $R_bCO$ or $R_bSO_2$- where $R_a$ and $R_b$ are alkyl, cycloalkyl, aryl, heterocyclic, heterocyclic alkyl, heterocyclic aryl, aryloxy alkyl, heterocyclic aryloxy alkyl, aryl alkyl, heterocyclic aryl alkyl, heterocyclic oxyalkyl, and $R_a$ and $R_b$ may be substituted with up to three members selected from amine, carboxy, alkoxy carbonyl hydroxy, alkyl, halo and alkoxy groups.

E is 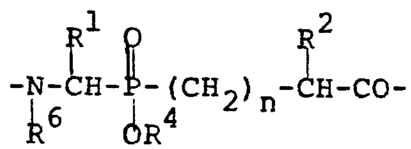 ;

G is $-R^3$ or is 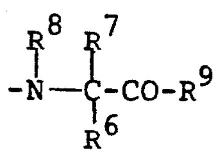 ,

wherein

$R^1$ is alkyl, alkenyl, alkynyl, cycloalkyl, cycloalkenyl, cycloalkyl alkyl, aryl alkyl, heterocyclic, heterocyclic alkyl, heterocyclic aryl alkyl, each of which may be substituted by alkyl, halo, amino and alkoxy groups.

n is 0, 1.

$R^2$ is hydrogen, alkyl, alkenyl, alkynyl, cycloalkyl, cycloalkenyl, cycloalkyl alkyl, aryl, aryl alkyl, heterocyclic, heterocyclic alkyl, each of which may be substituted by alkyl, hydroxy, halo, amino, alkylamino, dialkylamino, and alkoxy groups.

$R^3$ is OH, $NH_2$, $NHR^3_a$, $NR^3_aN^3_b$, $OR^3_c$ where $R^3_a$, $R^3_b$, and $R^3_c$ are separately alkyl alkenyl, alkynyl, cycloalkyl, cycloalkenyl, cycloalkyl alkyl, aryl, aryl alkyl, heterocyclic, heterocyclic alkyl, each of which may be substituted with up to three groups selected from amino, alkylamino, dialkyl amino, trialkyl ammonium, hydroxy, alkoxy, aryloxy, aryl alkoxyl, or halo, and $R^3_c$ is additionally $R^3_d$-CO-V-$CR^3_eR^3_f$ wherein $R^3_d$ is alkyl or aryl; $R^3_e$ and $R^3_f$ are hydrogen or alkyl; V is -O- or -NH-.

$R^4$ is hydrogen, alkyl alkenyl, alkynyl, aryl, arylalkyl, each of which may be substituted with amino, alkylamino, dialkylamino, trialkyl ammonium, hydroxy, alkoxy, halo or alkyl groups or $R^4_a$-CO-V-$CR^4_bR^4_c$ wherein $R^4_a$ is alkyl or aryl; $R^4_b$ and $R^4_c$ are hydrogen or alkyl; and V is -O- or -NH-.

$R^6$ is H or methyl.

$R^7$ is hydrogen, alkyl alkenyl, alkynyl, cycloalkyl cycloalkenyl, aryl, cycloalkyl alkyl, aryl alkyl, heterocyclic, heterocyclic alkyl, aryloxy alkyl, heterocyclic oxy alkyl,

heterocyclic oxy, each of which may be substituted with up to three groups selected from amino, alkyl amino, dialkyl amino, trialkyl ammonium, hydroxy, alkoxy, carboxy, alkoxycarbonyl, alkylthio, arylthio, thiol, guanidino, carboxamido and alkanoylamino groups.

and when taken together with $NR^8$ may be a cyclic amino acid of formulas:

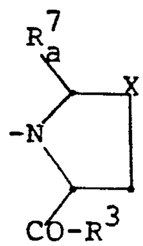

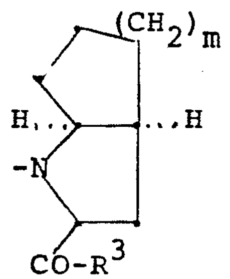

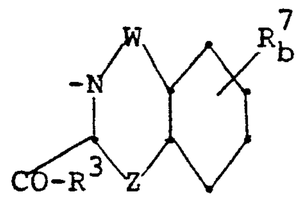

where $R_a^7$ is hydrogen, phenyl, hydroxyphenyl; X is -S- or $-CH_2-$ or $-CH-R_b^7$; m is 1 or 2; and $R_b^7$ is cyclohexyl, phenylthio; W and Z are bonds or $-CH_2-$, and $R^3$ is as defined above.

$R^8$ is hydrogen, methyl and cycloalkyl (including cyclopentyl and indanyl) such that when $R^8$ is cycloalkyl, $R^6$ and $R^7$ are hydrogen.

$R^9$ is hydroxy, $OR_a^3$, $-NH_2$, $-NHR_a^3$, $NR_a^3R_b^3$, where $R_a^3$ and $R_b^3$ are as defined above such that when A is absent, $R^9$ can be 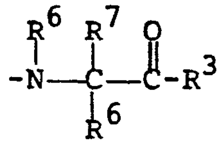 where $R^3$, and $R^6$ and $R^7$ are as defined above.

and pharmaceutically acceptable salts thereof.

2. A compound of Claim 1 selected from:

1. (N-carbobenzoxy-1-amino-2-cyclohexylethyl) 2-carboxy-4-methylpentylphosphinic acid
2. (N-carbobenzoxy-1-amino-2-cyclohexylethyl) 2-carboxy-3-methylbutylphosphinic acid
3. (N-t-butoxycarbonyl-1-amino-2-cyclohexylethyl) 2-carboxy-3-methylbutylphosphinic acid
4. (N-carbobenzoxy-1-amino-2-cyclohexylethyl) 2-carbomethoxy-4-methylpentylphosphinic acid
5. (N-carbobenzoxy-1-amino-2-cyclohexylethyl) 2-carboxamido-4-methylpentylphosphinic acid
6. (N-t-butoxycarbonyl-1-amino-2-cyclohexylethyl) 2-(N-benzyl)carboxamido-3-methylbutylphosphinic acid
7. [N-(2-phenyl)ethyl-1-amino-2-cyclohexylethyl] 2-carboxy-4-methylpentylphosphinic acid
8. [N-(N-benzyl)carboxamidomethyl-1-amino-2-cyclohexylethyl] 2-carboxy-3-methylbutylphosphinic acid
9. [N-(1-carboethoxy-2-phenylethyl)-1-amino-2-cyclohexylethyl] 2-carboxy-3-methylbutylphosphinic acid
10. (N-carbobenzoxy-1-amino-2-cyclohexylethyl)carbomethoxymethylphosphinic acid
11. (N-carbobenzoxy-1-amino-2-cyclohexylethyl)carboxymethylphosphinic acid
12. (N-carbobenzoxy-1-amino-2-cyclohexylethyl) carboxamidomethylphosphinic acid
13. (N-carbobenzoxy-1-amino-2-cyclohexylethyl) 2-carboxy-3-cyclohexylpropylphosphinic acid
14. [N-(3-phenyl)propionyl-1-amino-2-cyclohexylethyl] 2-carboxy-4-methylpentylphosphinic acid
15. (N-phenoxyacetyl-1-amino-2-cyclohexylethyl) 2-carboxy-3-methylbutylphosphinic acid

16. [N-(4-amino)butanoyl-1-amino-2-cyclohexylethyl] 2-carboxy-3-methylbutylphosphinic acid
17. (N-naphthyloxyacetyl)-1-amino-2-cyclohexylethyl) 2-carboxy-3-methylbutylphosphinic acid
18. Methyl (N-carbobenzoxy-1-amino-2-cyclohexylethyl) 2-carboxy-4-methylpentylphosphinate
19. Methyl (N-carbobenzoxy-1-amino-2-cyclohexylethyl)-carbomethoxymethylphosphinate
20. Ethyl [N-(3-phenyl)propionyl-1-amino-2-cyclohexylethyl] 2-carbomethoxy-3-methylbutylphosphinate
21. [N-(1-carboethoxy-5-aminopentyl)-1-amino-2-cyclohexylethyl] 2-carboxy-3-methylbutylphosphinic acid
22. [N-(1-carbobenzoxy-5-aminopentyl)-1-amino-2-cyclohexylethyl] 2-carboethoxy-3-methylbutylphosphinic acid
23. [N-carbobenzoxy-1-amino-2-cyclohexylethyl] 2-(N-(m-aminomethyl)benzyl)carboxamido-3-methylbutylphosphinic acid

3. A compound of Claim 1 selected from:

24. (N-CBZ-1-amino-2-cyclohexylethyl) 2-carboxy-4-methylpentylphosphinic acid
25. (N-CBZ-1-amino-2-cyclohexylethyl) 2-carbomethoxy-4-methylpentylphosphinic acid
26. Methyl (N-CBZ-1-amino-2-cyclohexylethyl) 2-carbomethoxy-4-methylpentylphosphinate
27. Methyl (N-BOC-1-amino-2-cyclohexylethyl) 2-carbomethoxy-4-methylpentylphosphinate
28. (1-Amino-2-cyclohexylethyl) 2-carbomethoxy-4-methylpentylphosphinic acid hydrobromide
29. Methyl (1-amino-2-cyclohexylethyl) 2-carbomethoxy-4-methylpentylphosphinate hydrobromide

30. Trimethylacetoxymethyl (N-CBZ-1-amino-2-cyclohexylethyl) 2-carbomethoxy-4-methylpentylphosphinate
31. Methyl (N-BOC-1-amino-2-cyclohexylethyl) 2-carbomethoxy-4-methyl-(E)-2-pentenylphosphinate
32. Methyl (N-BOC-1-amino-2-cyclohexylethyl) 2-carbomethoxy-4-methyl-(Z)-2-pentenylphosphinate
33. (N-BOC-1-Amino-2-cyclohexylethyl) 2-carboxy-4-methyl-(E)-2-pentenylphosphinic acid
34. (N-BOC-1-Amino-2-cyclohexylethyl) 2-carboxy-4-methyl-(Z)-2-pentenylphosphinic acid
35. Methyl (N-CBZ-1-amino-2-cyclohexylethyl) 2-carbomethoxy-3-cyclohexylphosphinate
36. Methyl (N-CBZ-1-amino-2-cyclohexylethyl) carbomethoxymethylphosphinate
37. (1-Amino-2-cyclohexylethyl) carbomethoxymethylphosphinic acid
38. Methyl (1-amiho-2-cyclohexylethyl)carbomethoxymethylphosphinate hydrochloride
39. Methyl (N-CBZ-1-amino-3-methylbutyl)carbomethoxymethylphosphinate
40. Methyl (N-CBZ-1-amino-2-phenylethyl) 2-carbomethoxy-3-phenylpropylphosphinate
41. (1-Amino-2-phenylethyl) 2-carbomethoxy-3-phenylpropylphosphinate

4. A pharmaceutical composition for treating hypertension containing a compound of Claim 1.

5. A pharmaceutical composition for treating hypertension containing a compound of Claim 1 and another antihypertensive agent.